# EUROPÄISCHE PATENTSCHRIFT

(11) **EP 3 796 973 B1**
(45) Veröffentlichungstag und Bekanntmachung des Hinweises auf die Patenterteilung: **19.07.2023**
(21) Anmeldenummer: 20760765.6
(22) Anmeldetag: 13.08.2020
(51) Int. Cl.: A61N 5/06

(54) **LICHTAPPLIKATOR**
LIGHT-DELIVERY UNIT
UNITÉ DE DISTRIBUTION DE LUMIÈRE

(30) Priorität: 14.08.2019 DE 102019212199
(43) Veröffentlichungstag der Anmeldung: 31.03.2021
(73) Patentinhaber: Richard Wolf GmbH, 75438 Knittlingen (DE)
(72) Erfinder: WEBER, Bernd Claus, 76228 Karlsruhe (DE); WIRTH, Manfred, 01326 Dresden (DE)
(74) Vertreter: Patentanwälte Vollmann Hemmer Lindfeld Partnerschaft mbB
(86) Internationale Anmeldenummer: PCT/DE2020/200069
(87) Internationale Veröffentlichungsnummer: WO 2021/027998

(56) Entgegenhaltungen:
- US-A1- 2014 188 035
- US-A1- 2016 000 309
- US-A1- 2016 151 639
- US-A1- 2016 213 945

## Beschreibung

Die vorliegende Offenbarung betrifft einen Lichtapplikator zur Untersuchung und/oder Behandlung eines organischen Körpers, insbesondere zur photodynamischen Therapie (PDT) von pathologischem Gewebe.

Es ist bekannt, Endoskope dazu zu nutzen, um Videoaufnahmen vom Inneren eines menschlichen oder tierischen Körpers zu Zwecken der medizinischen Diagnose und/oder Therapie zu machen. Dabei ist es ein ständiges Bestreben, den Einführabschnitt von Endoskopen möglichst dünn auszugestalten, damit möglichst kleine Hohlräume eingesehen werden können und das Gewebe dabei möglichst wenig in Mitleidenschaft zu ziehen.

Endoskope werden allerdings nicht nur dazu genutzt, um Bild- oder Videoaufnahmen zu machen, sondern auch als Diagnose- oder Therapiemittel selbst eingesetzt. Die US 2016/000309 A1,

US 2016/051639 A1, US 2016/0213945 A1 und die US 2014/0188035 A1 beschreiben jeweils beispielsweise lichttherapeutische Mittel. Es ist bekannt, dass für die Detektion und Lokalisierung von prä- und frühmalignem Gewebe eine Fluoreszenz-Endoskopie eingesetzt werden kann, bei der es nicht auf eine natürliche Echtfarb-Darstellung des Gewebes ankommt, sondern lediglich auf eine Fluoreszenzanregung, mit der sich pathologisches Gewebe von gesundem Gewebe unterscheiden lässt. Dabei kann das mittels Lichtstrahlung angeregte pathologische Gewebe selbst oder eine auf pathologisches Gewebe hinweisende Bakterienansammlung spezifisch fluoreszieren und so gegenüber dem umliegenden gesunden Gewebe erkennbar lokalisiert werden. Die Fluoreszenz-Endoskopie kann beispielsweise im Rahmen einer photodynamischen Diagnose (PDD) und/oder photodynamischen

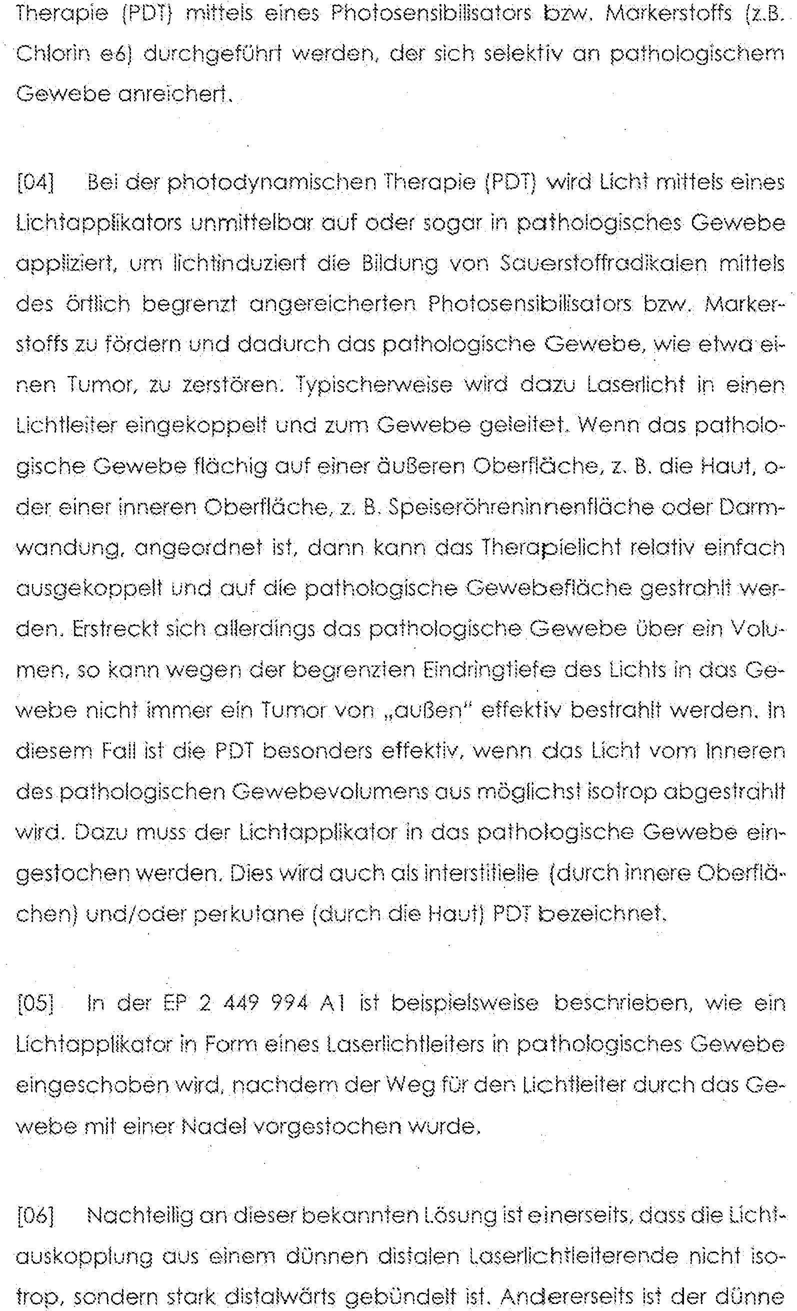

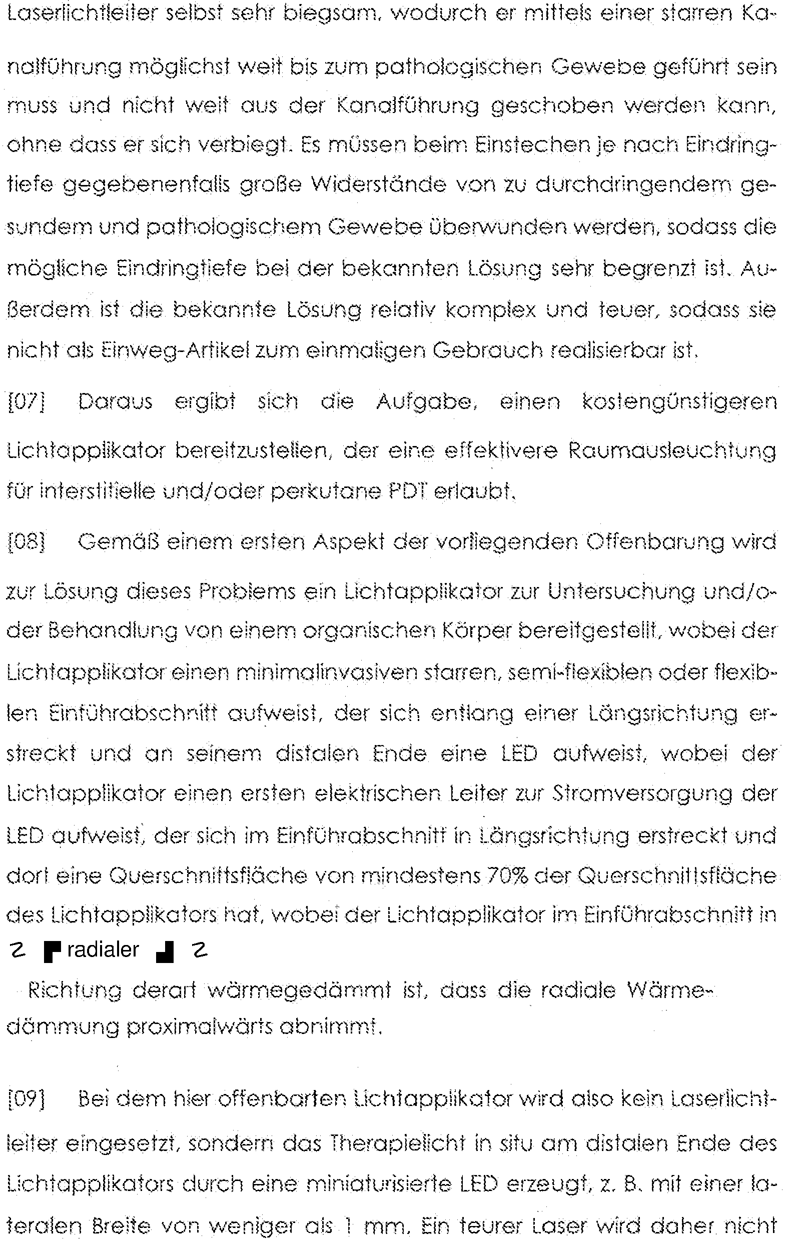

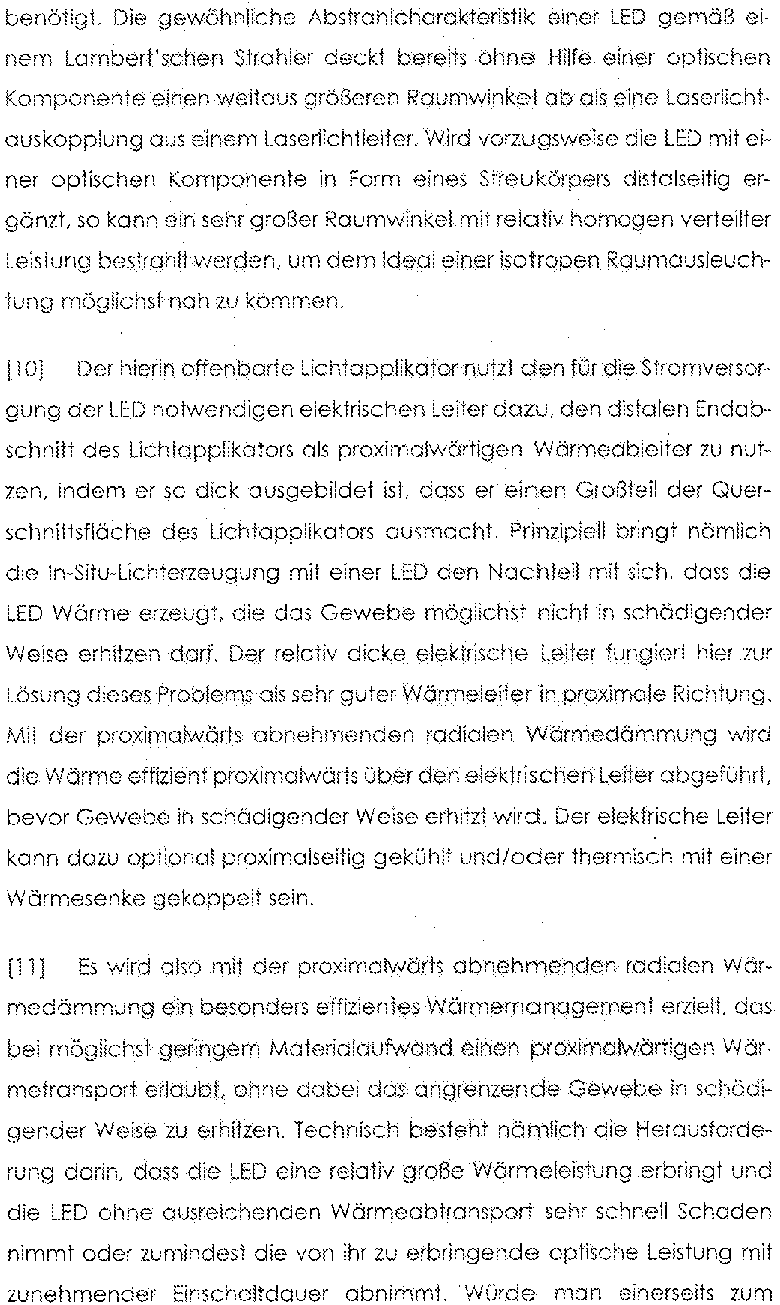

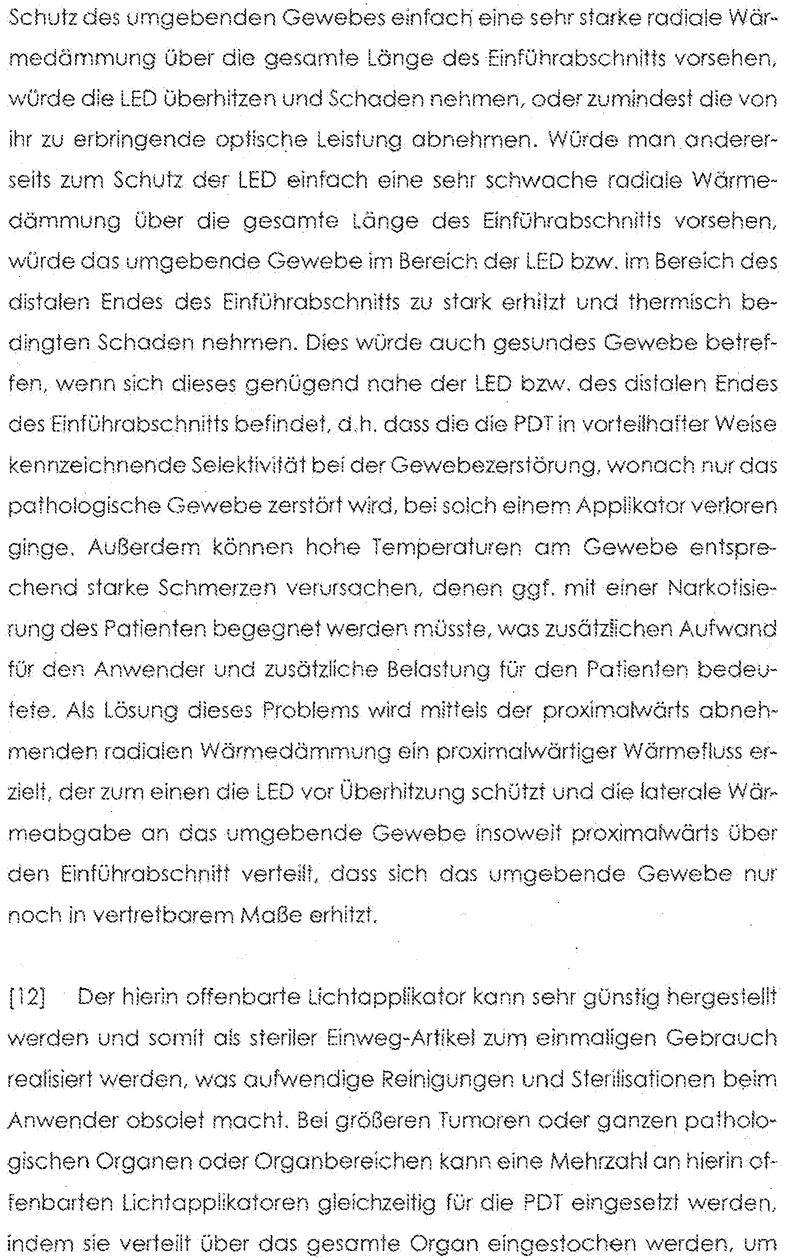

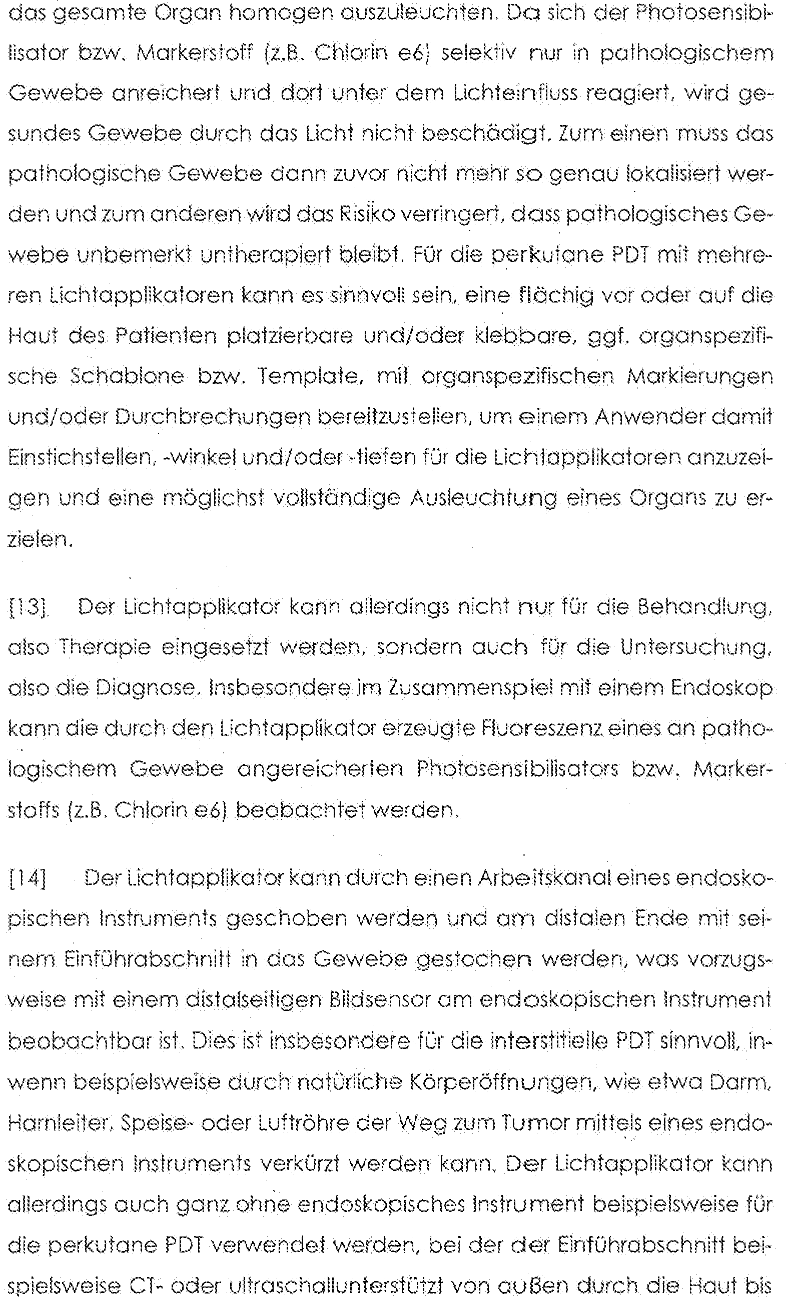

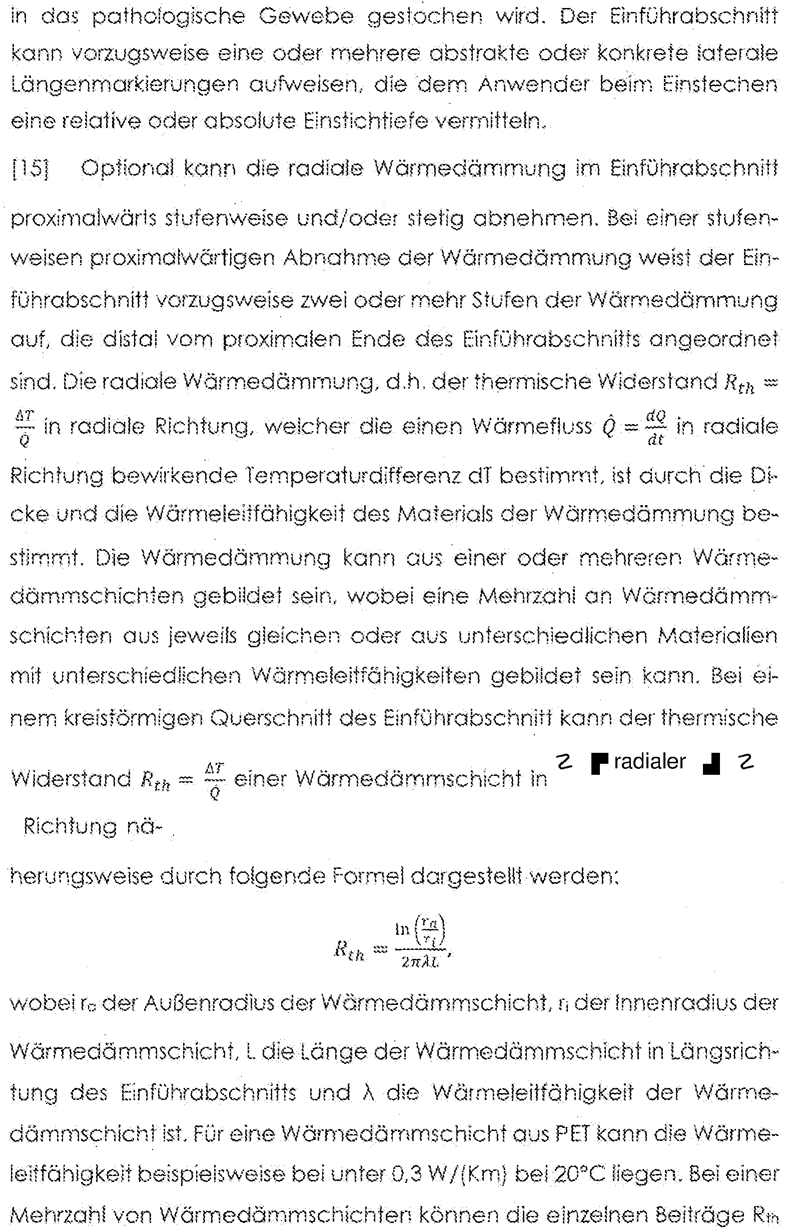

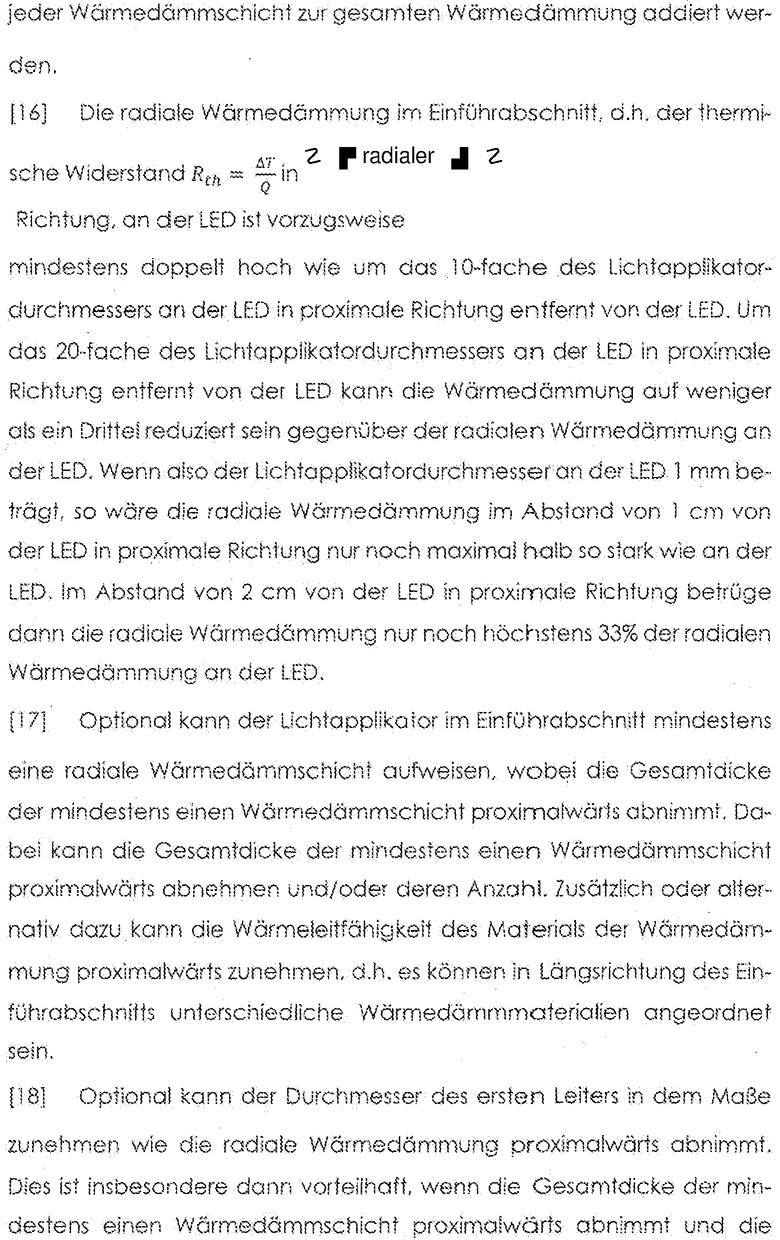

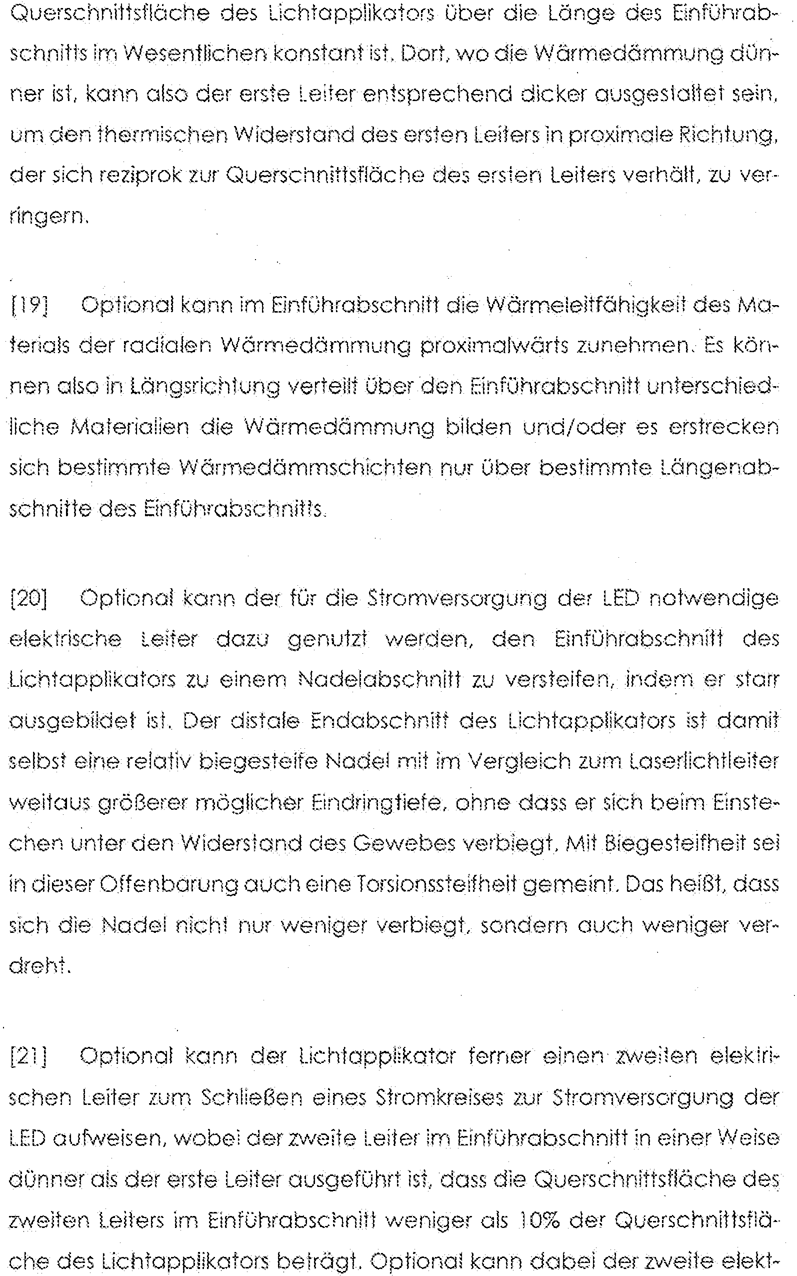

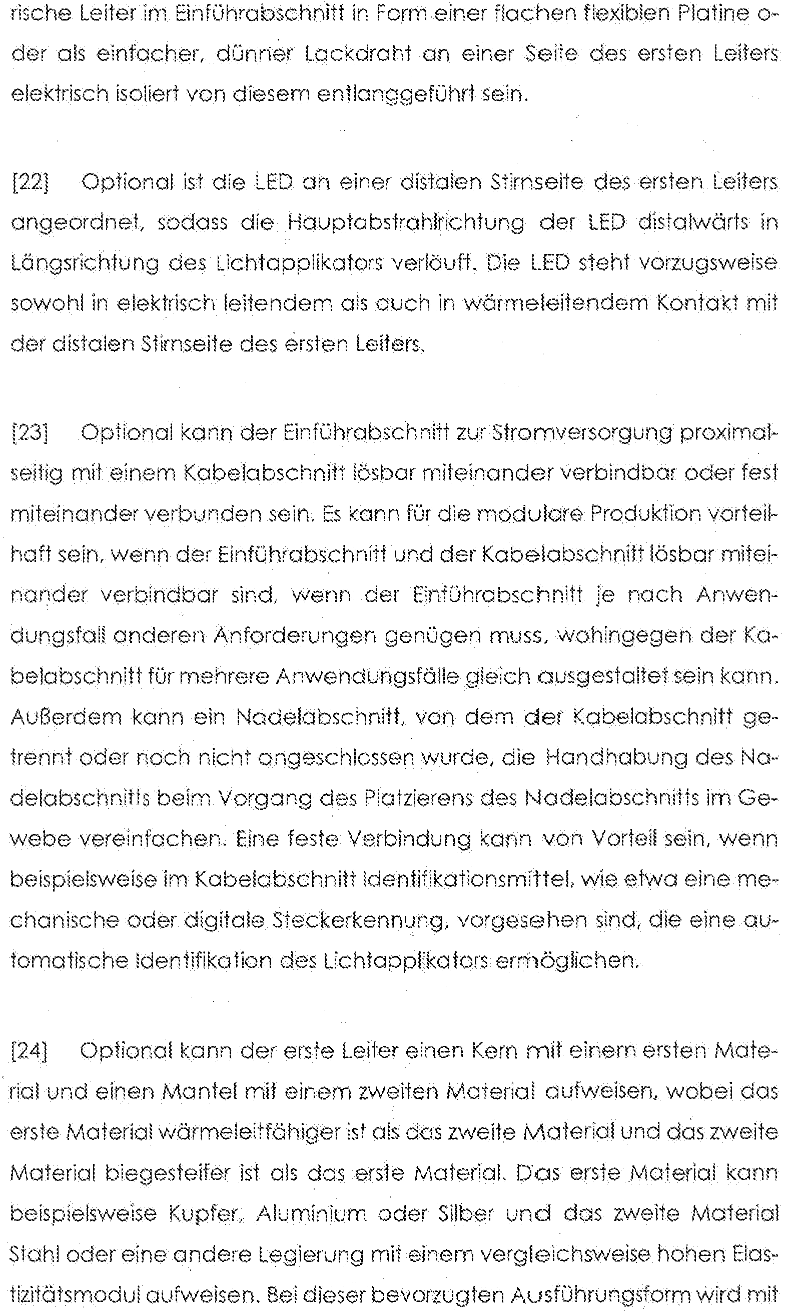

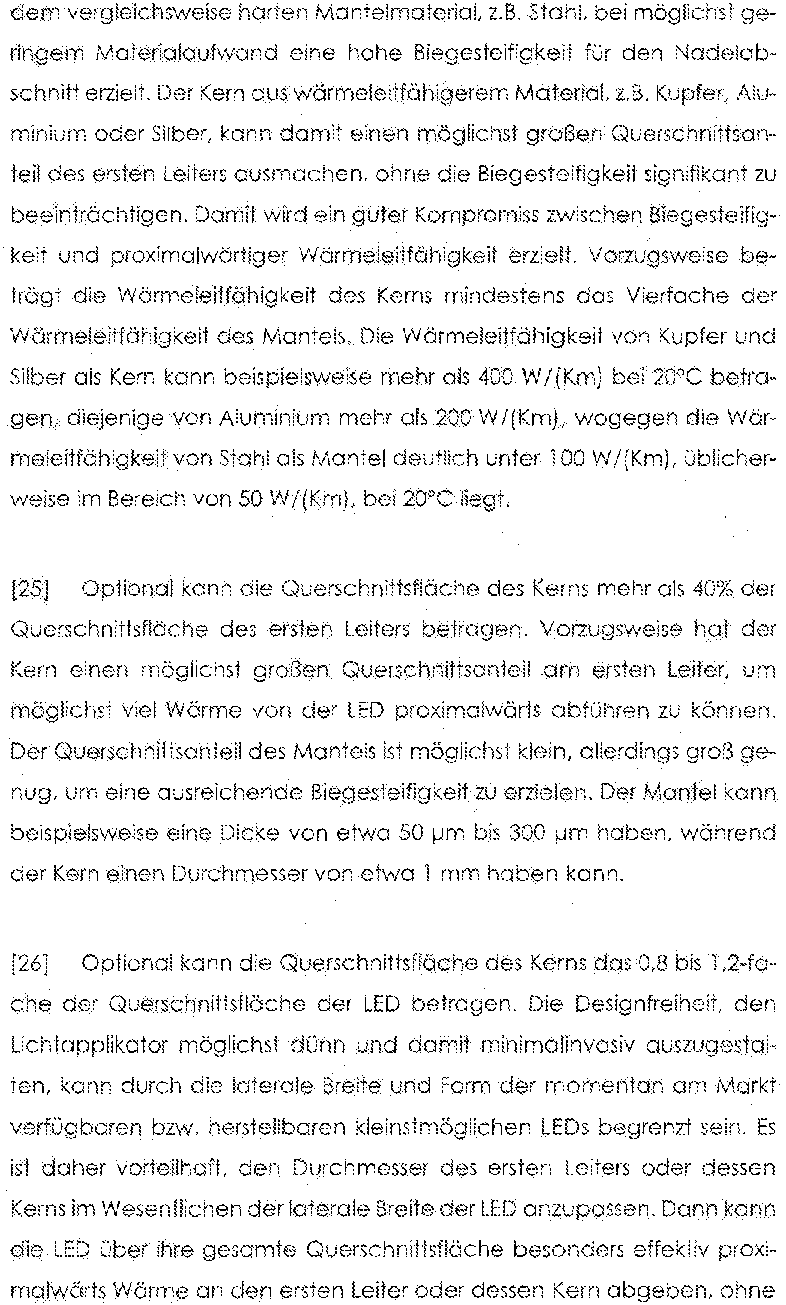

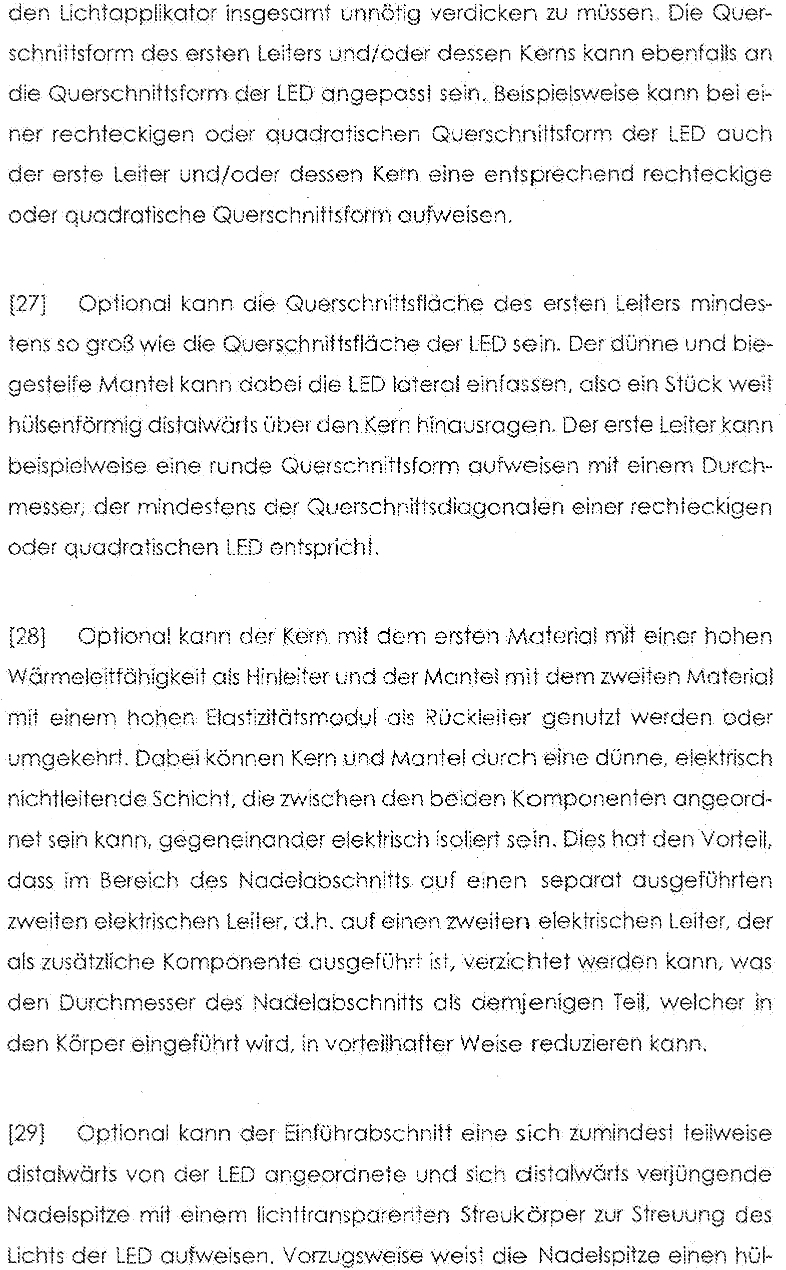

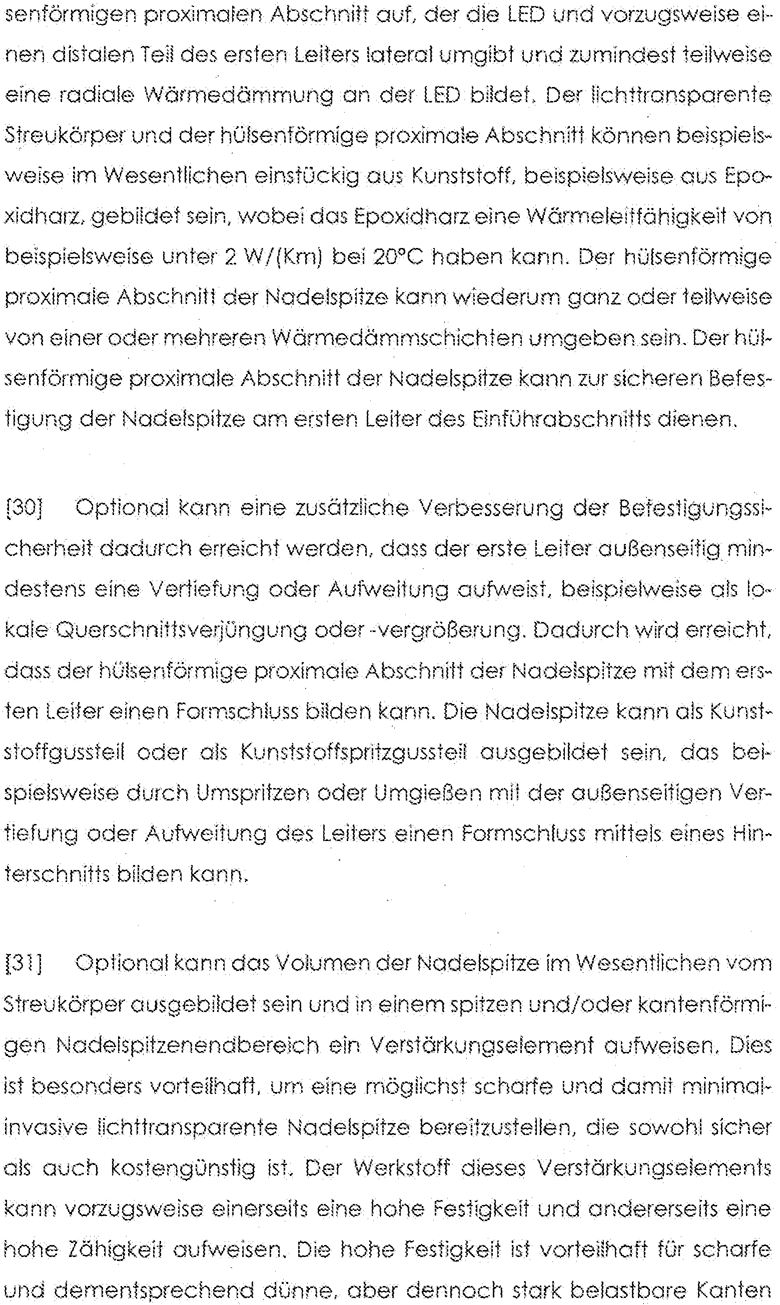

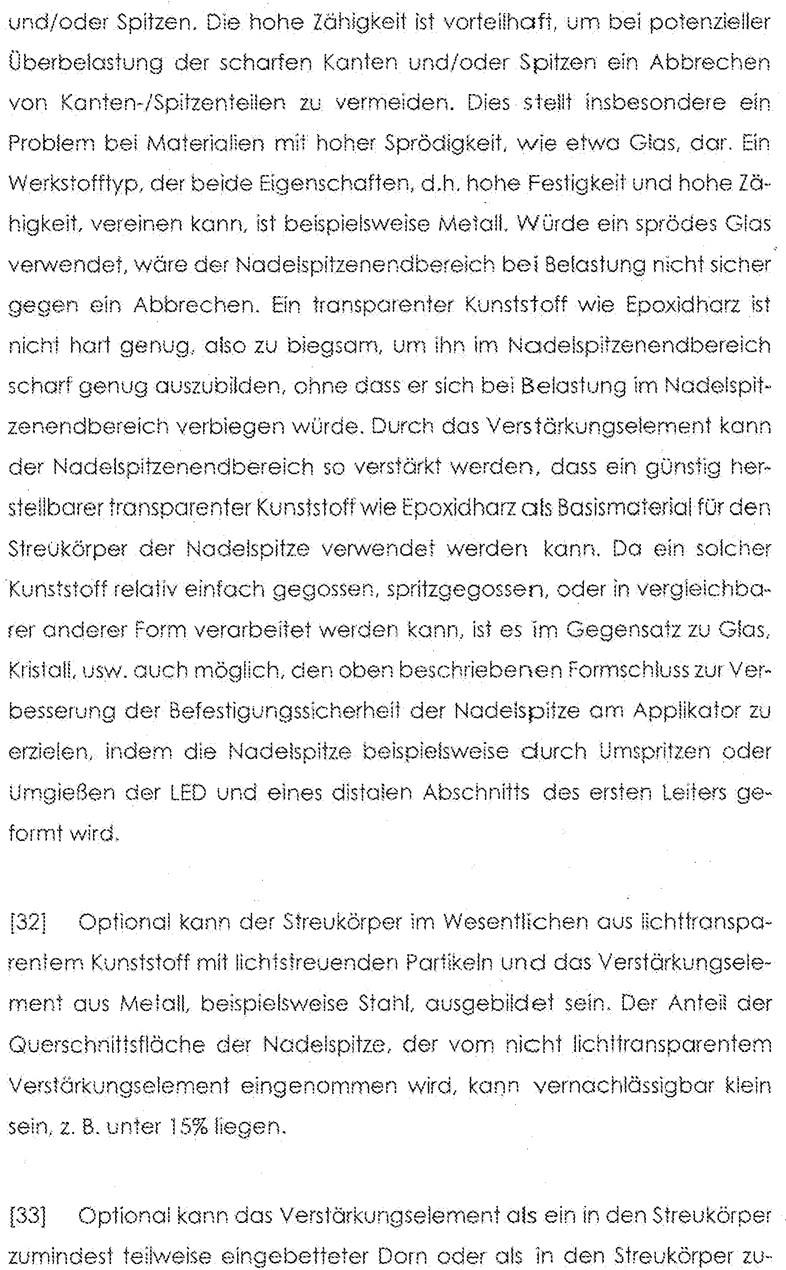

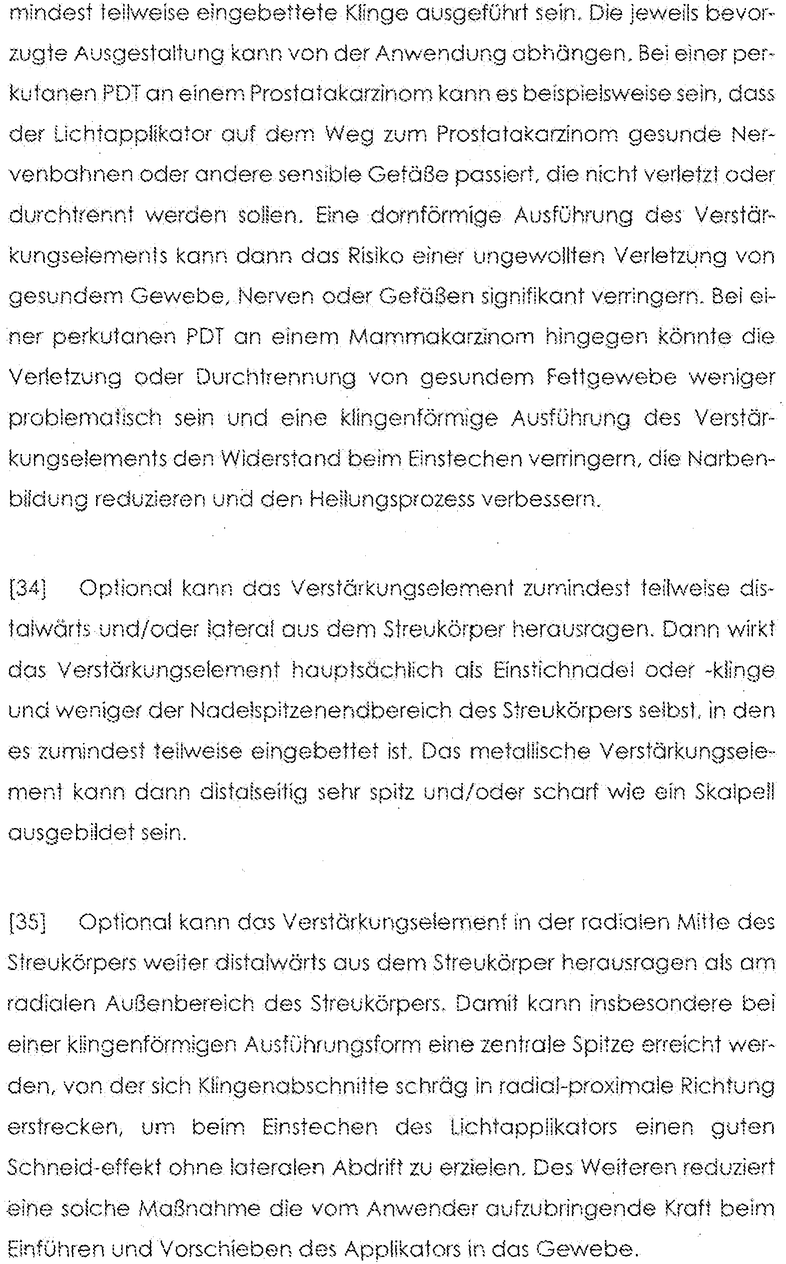

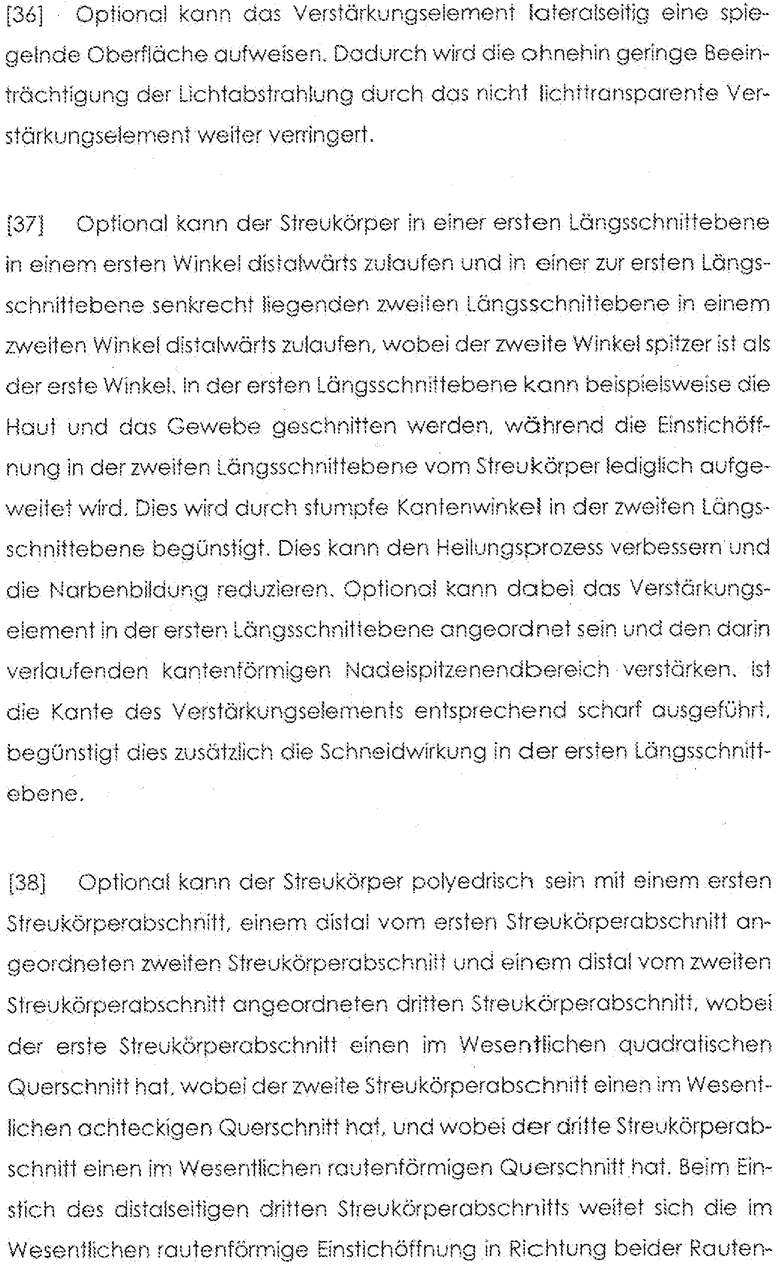

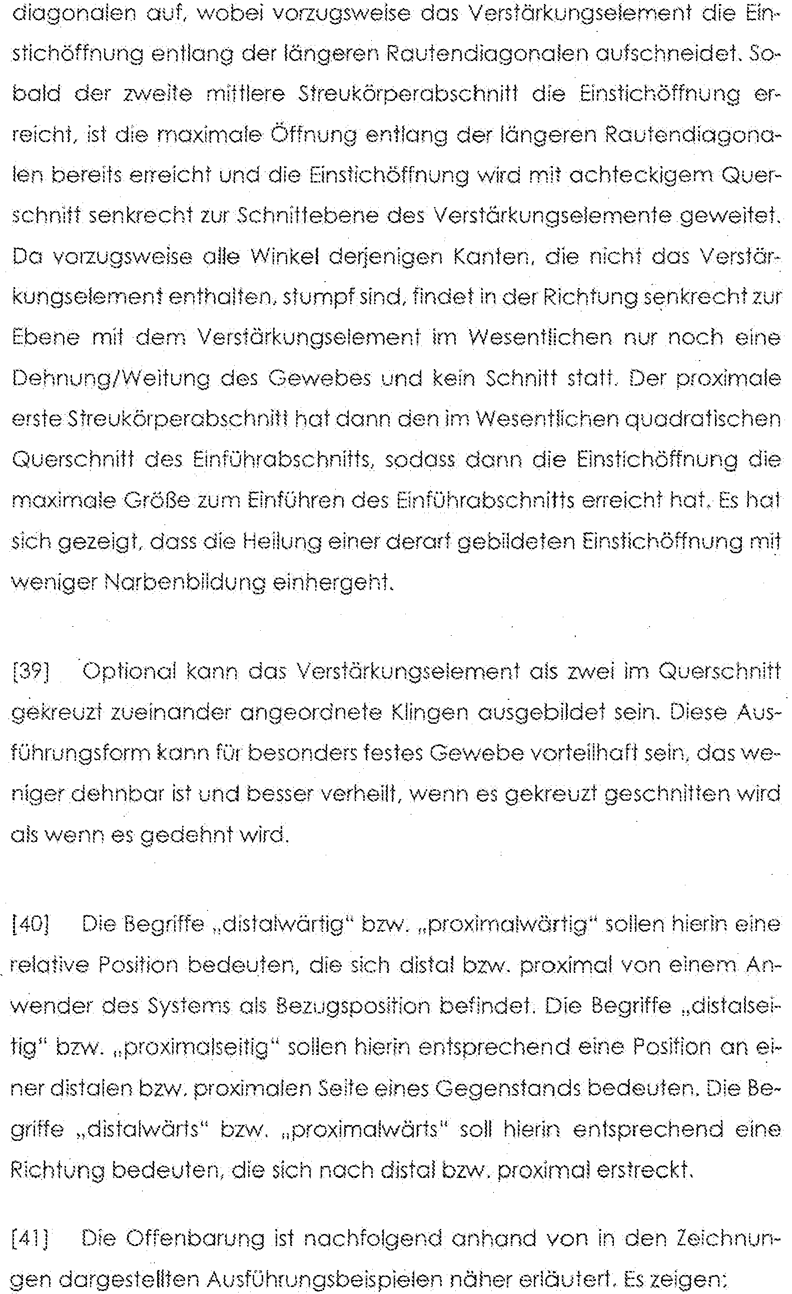

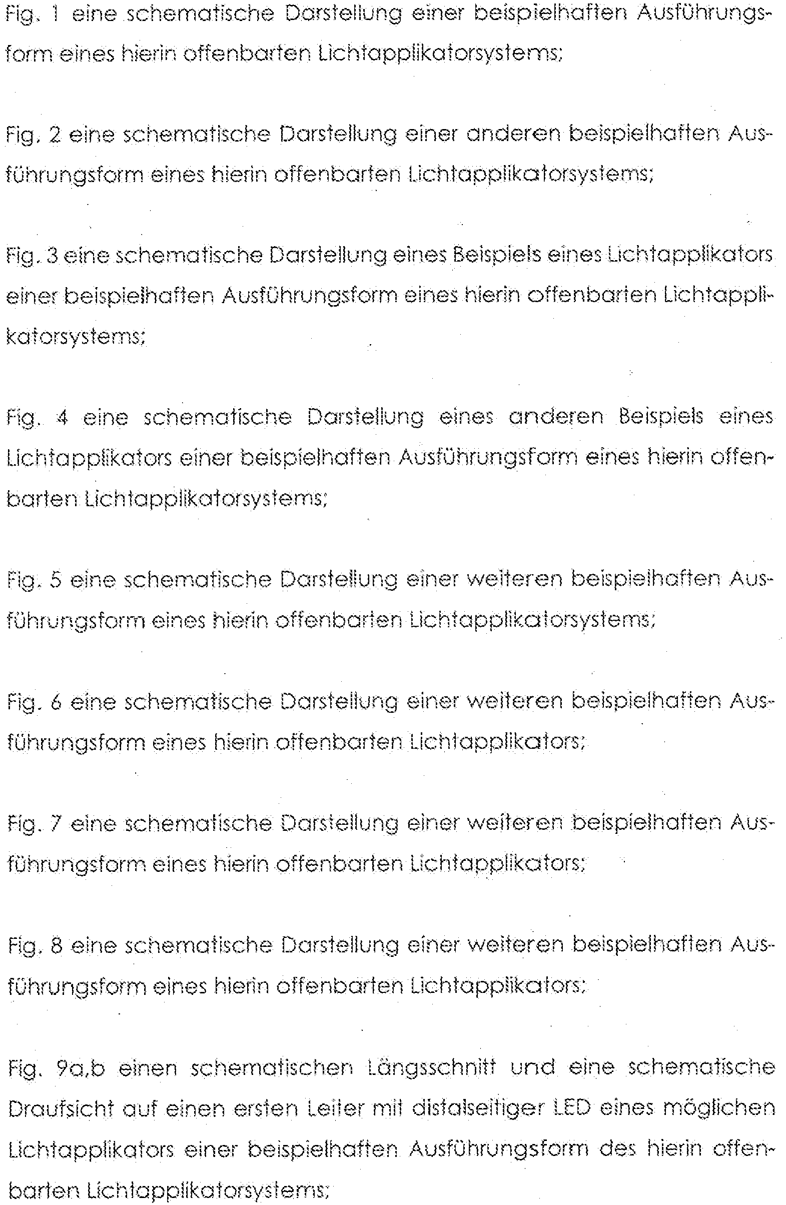

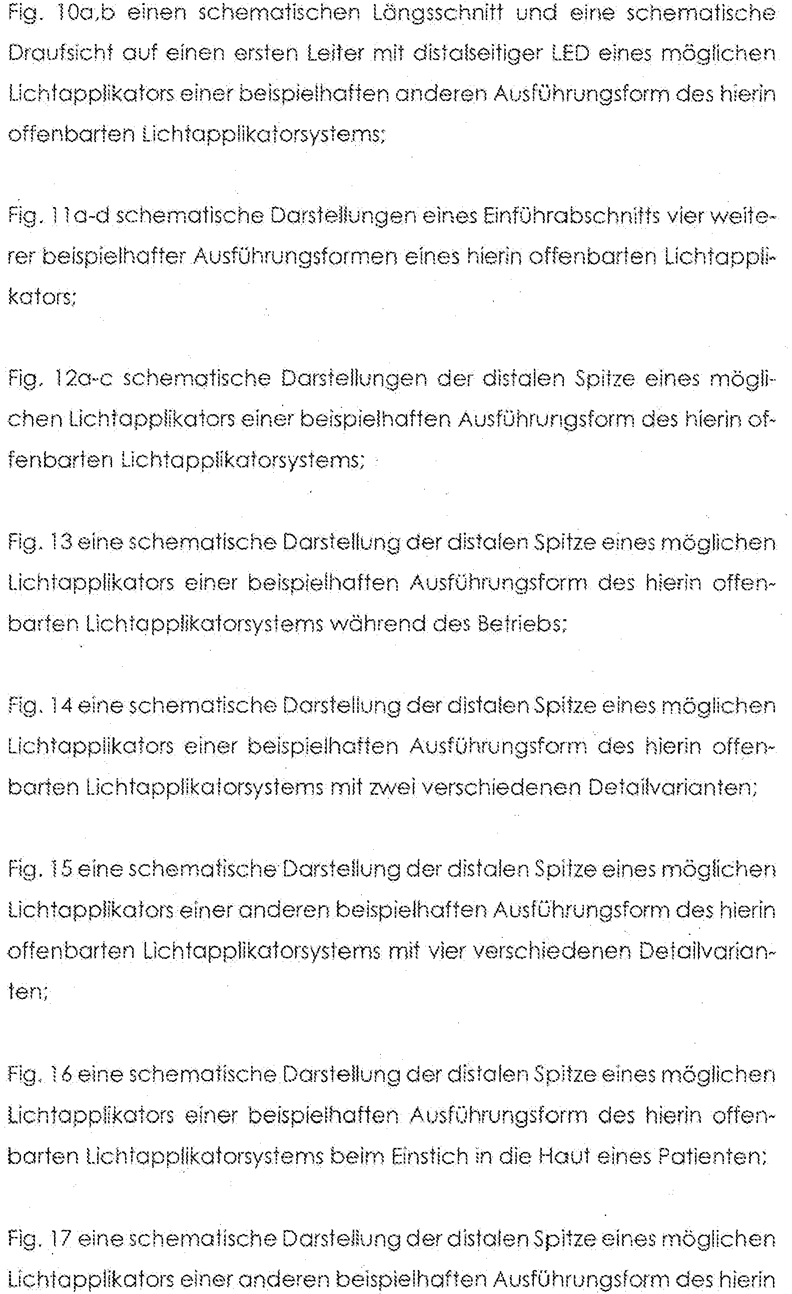

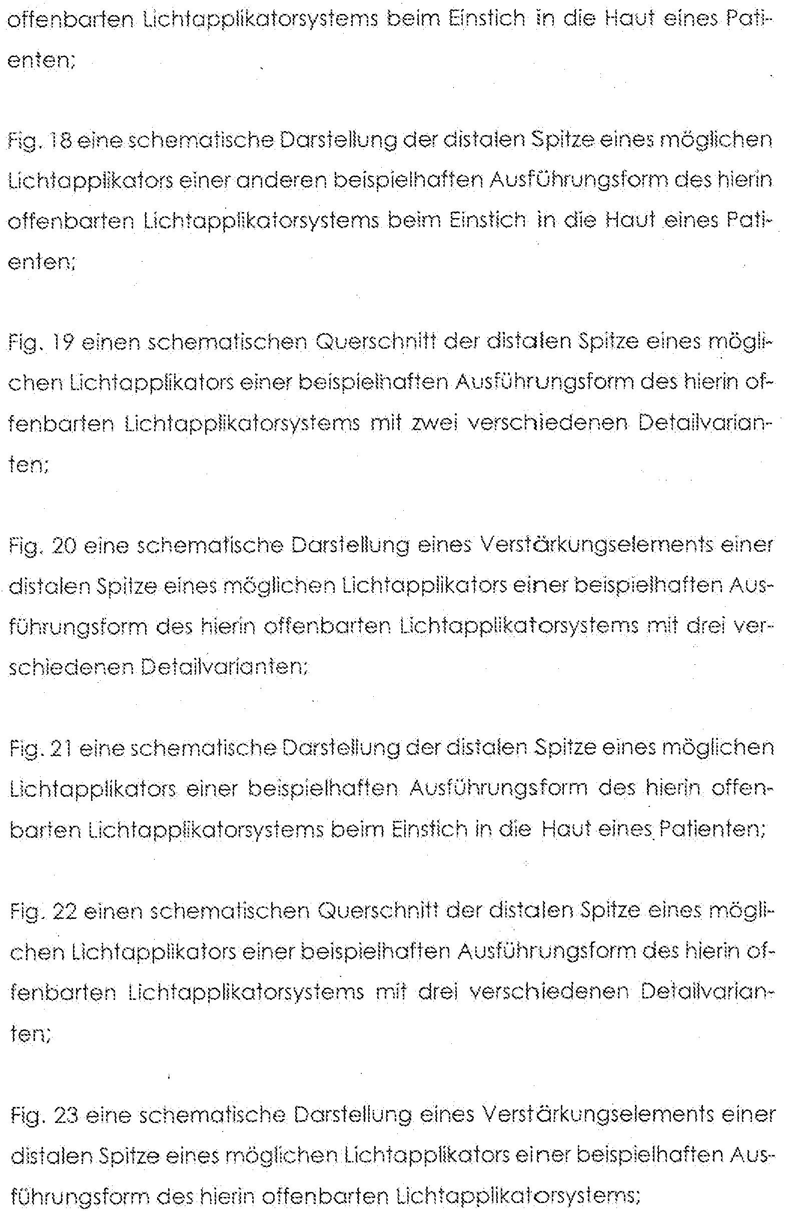

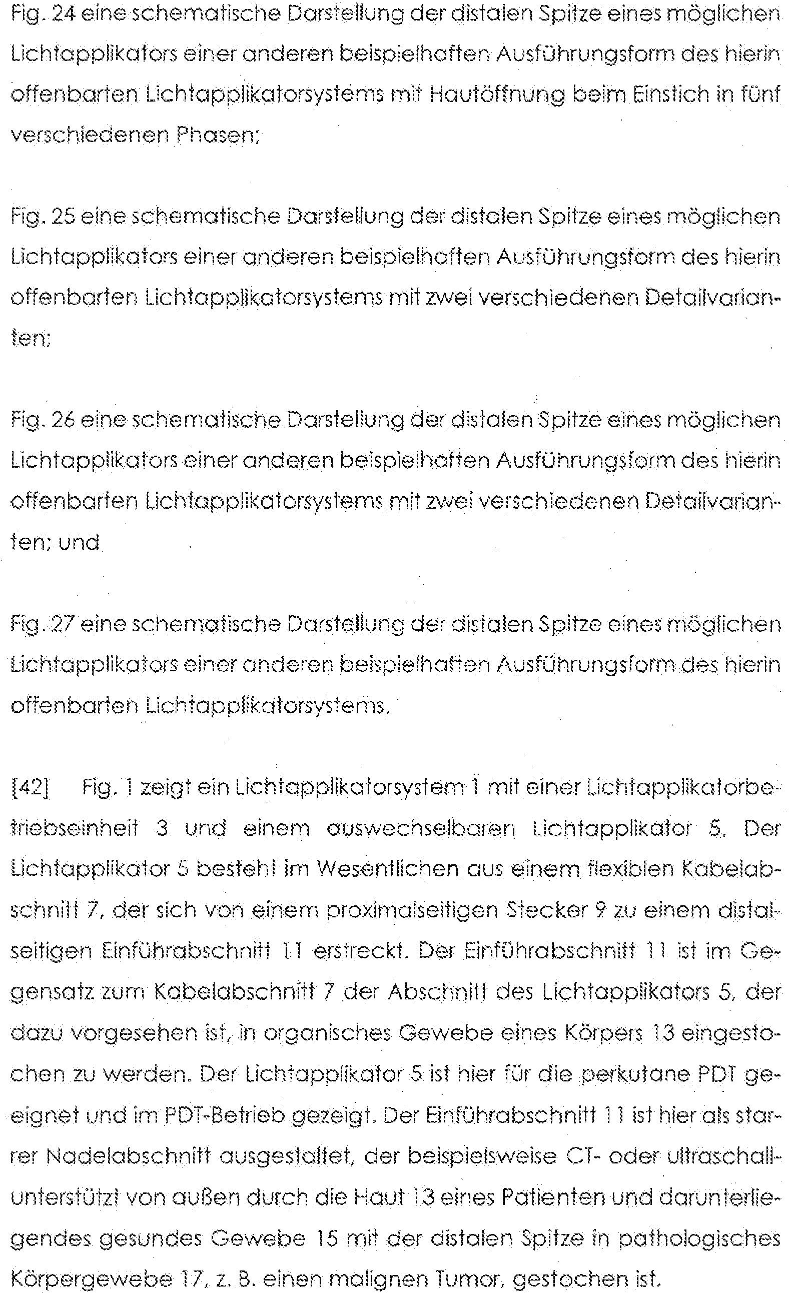

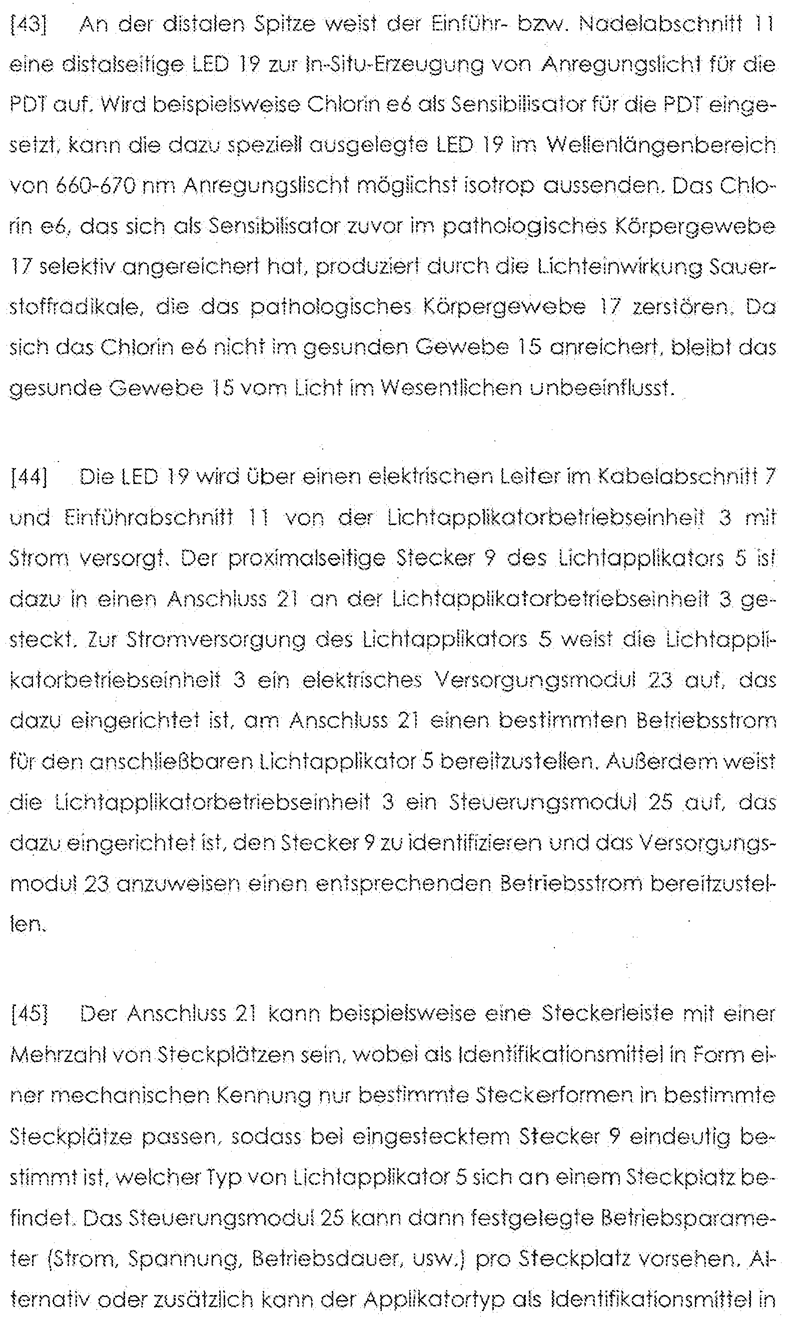

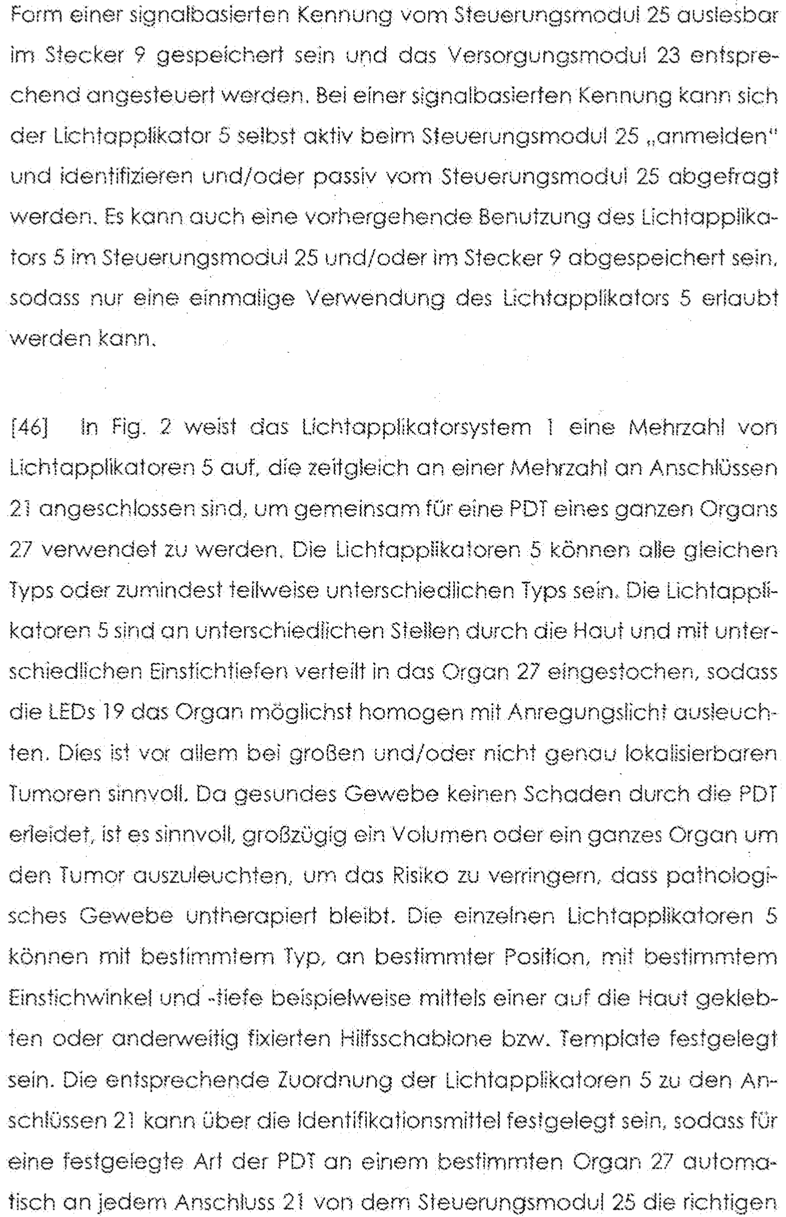

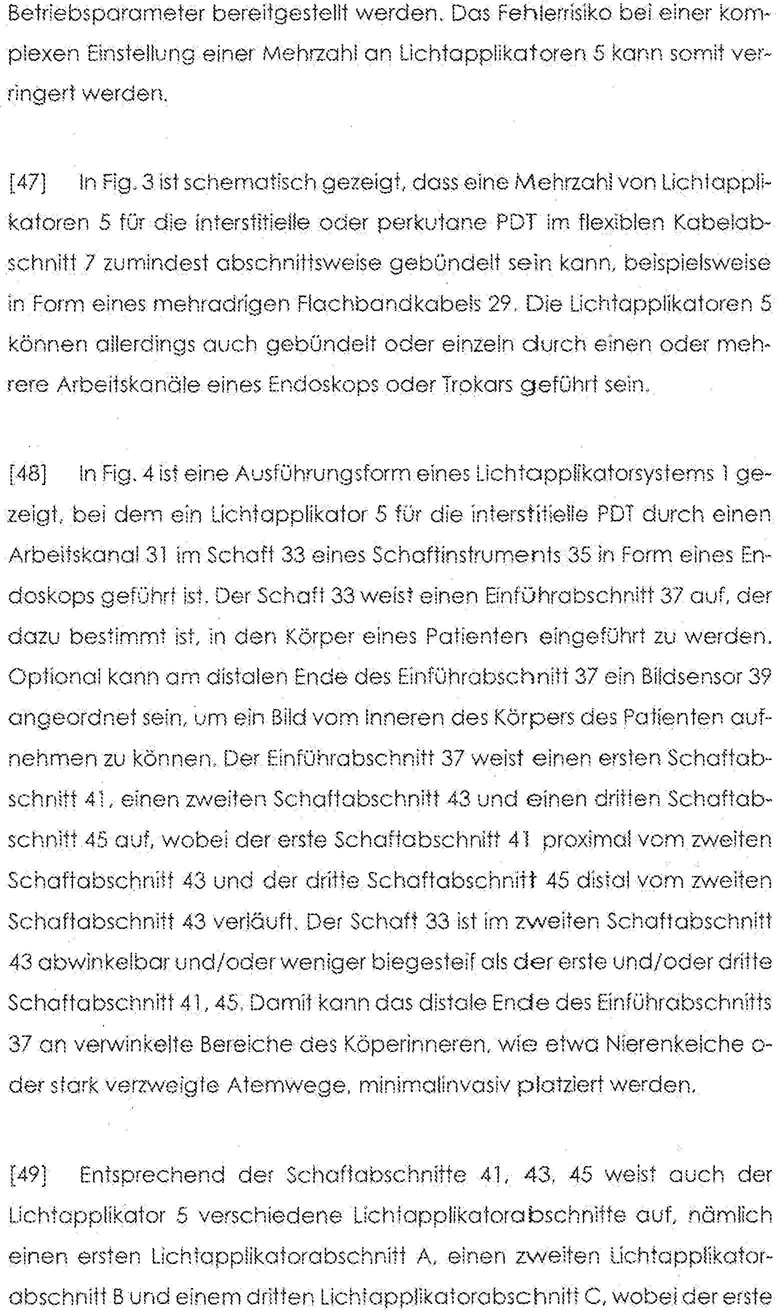

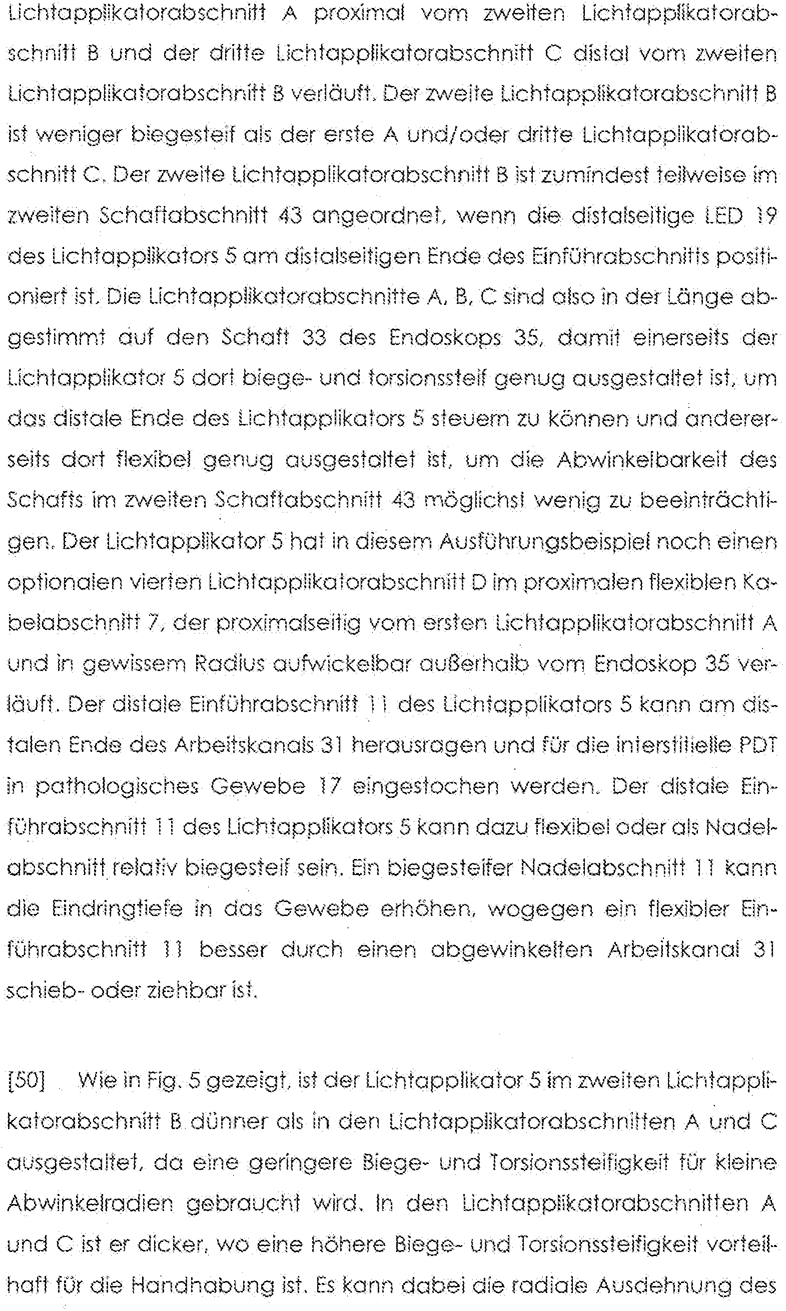

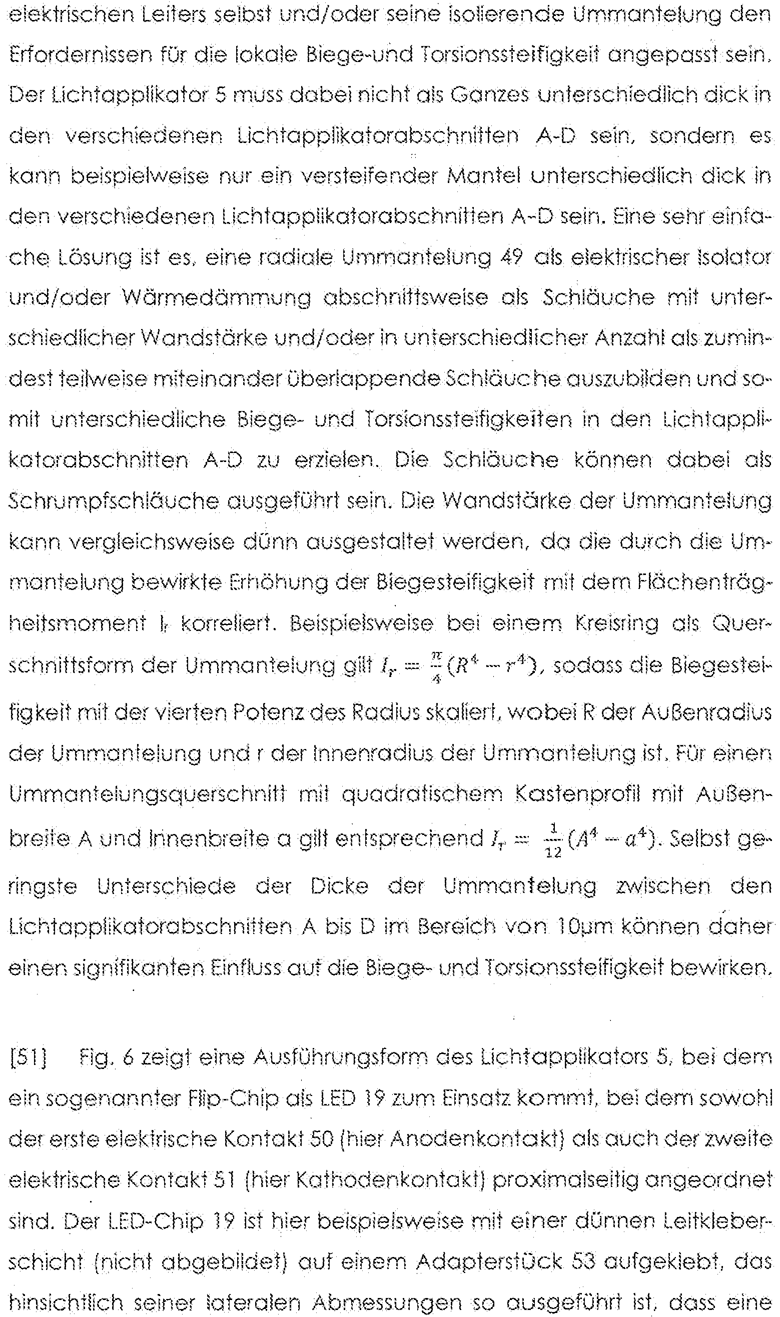

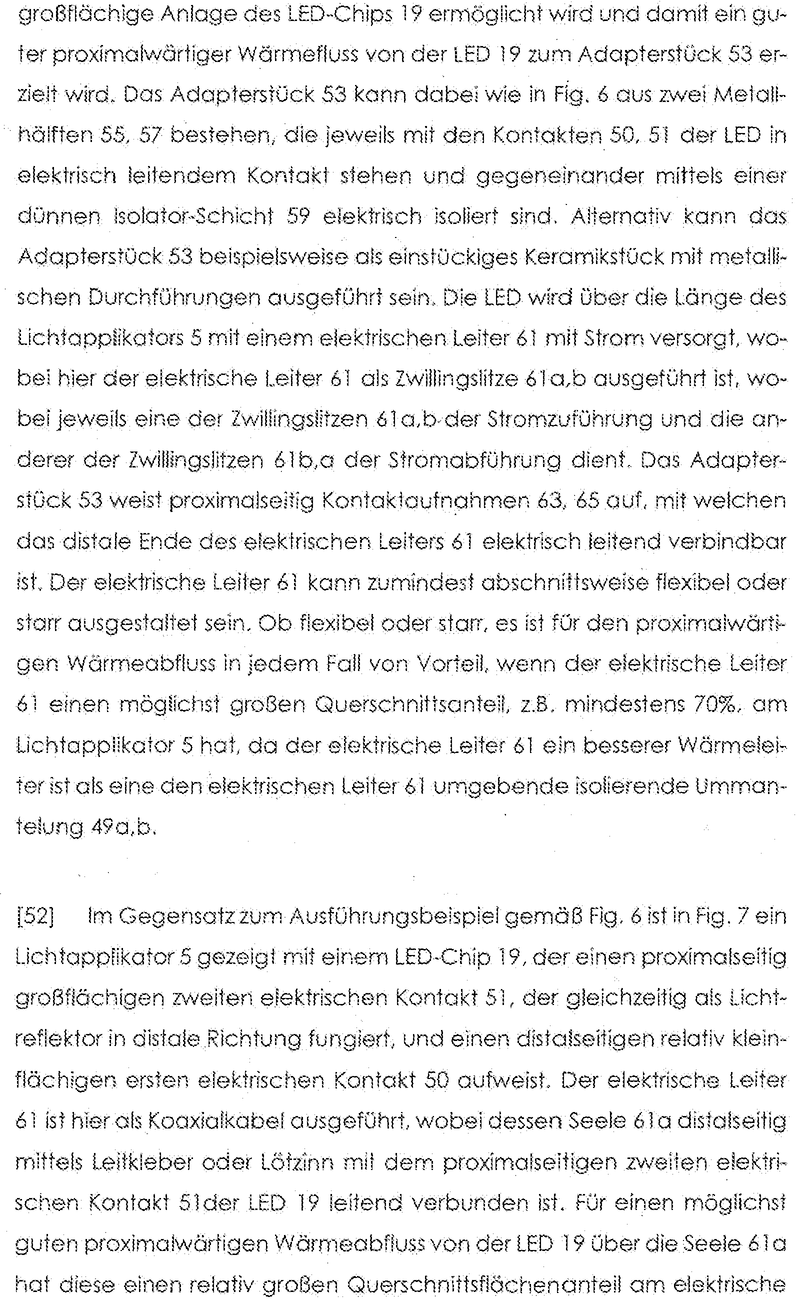

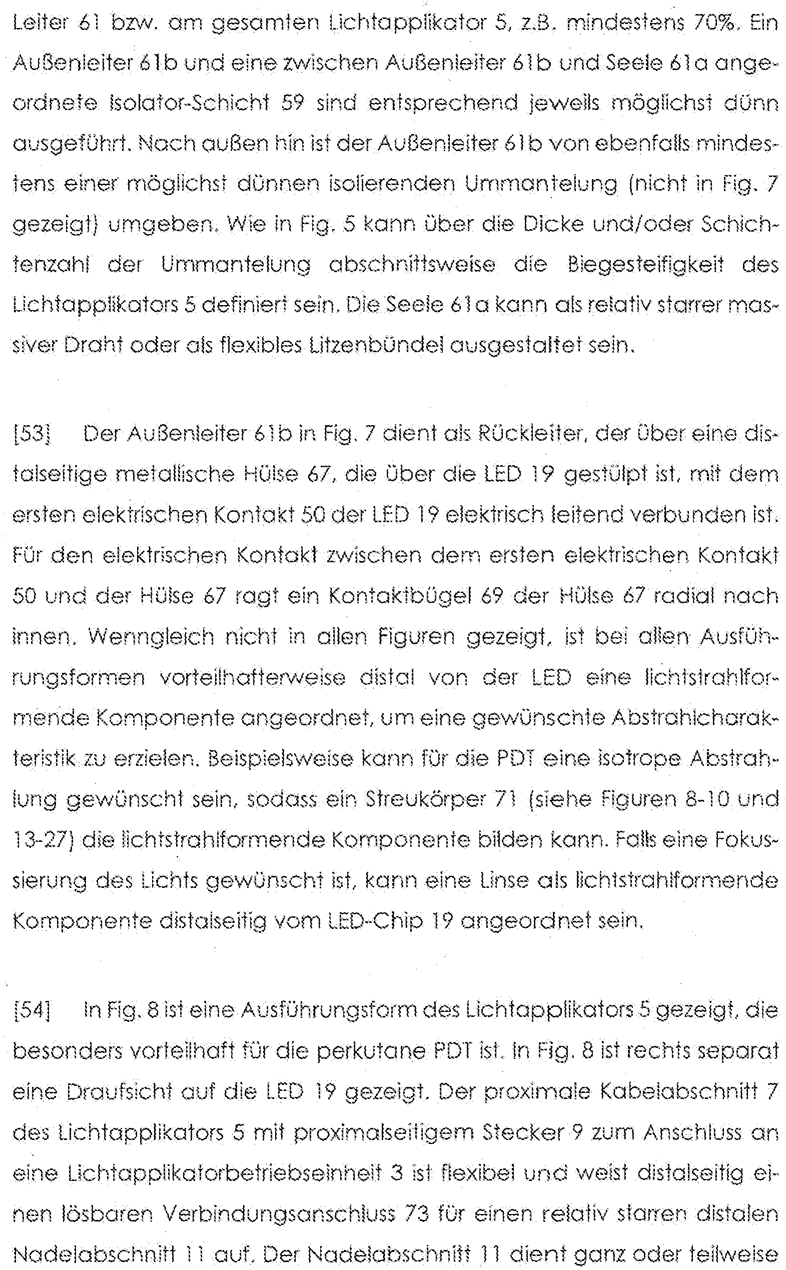

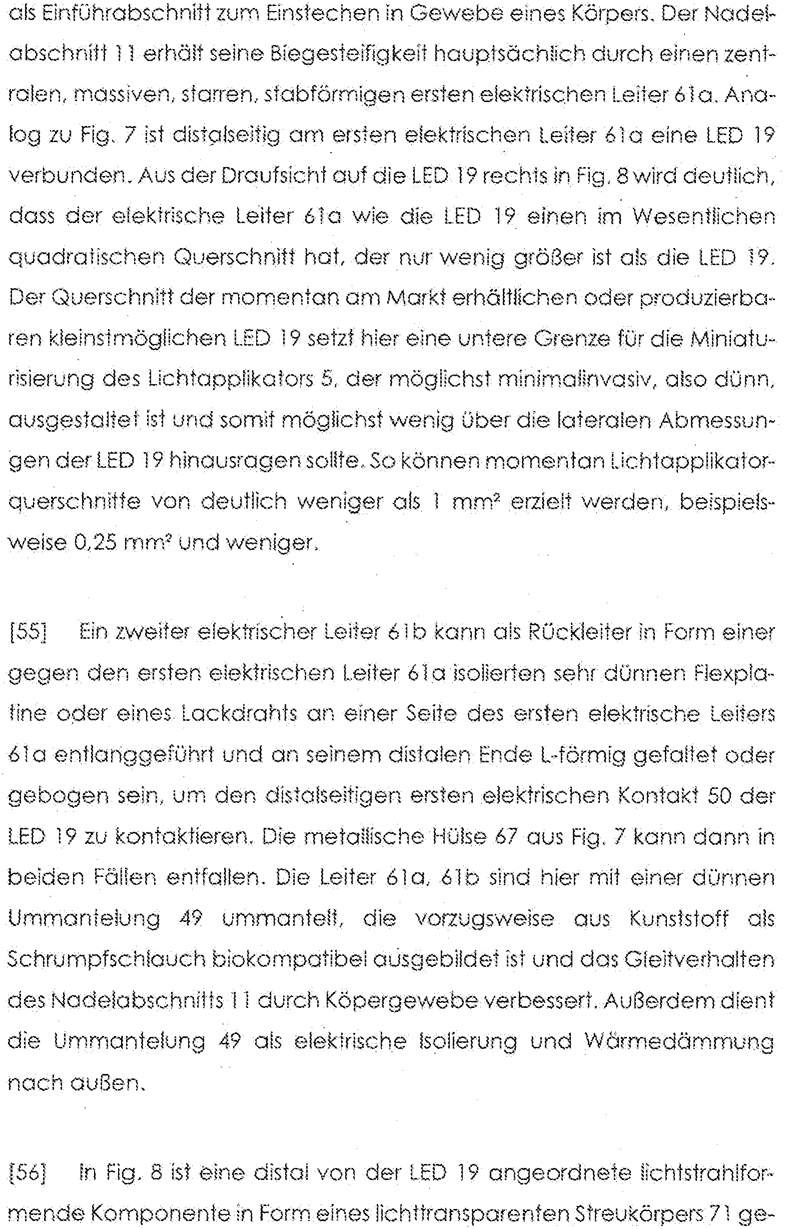

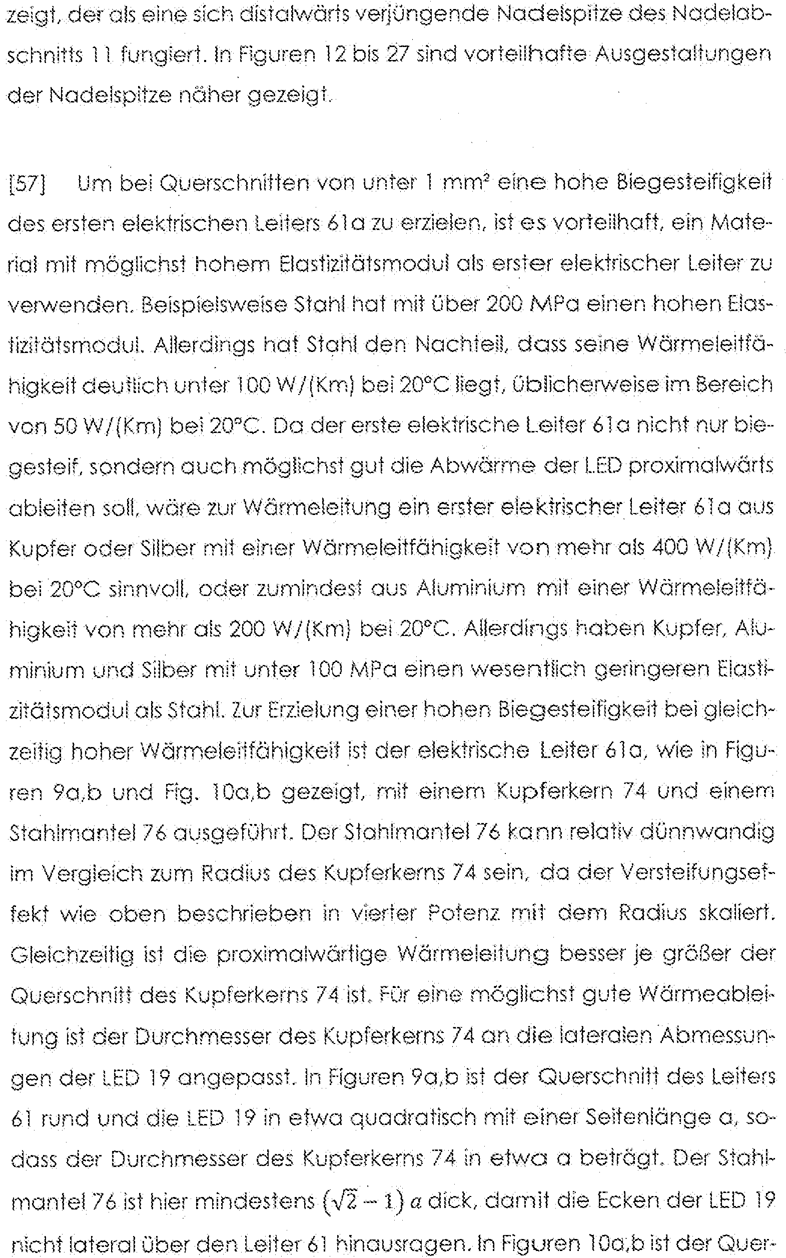

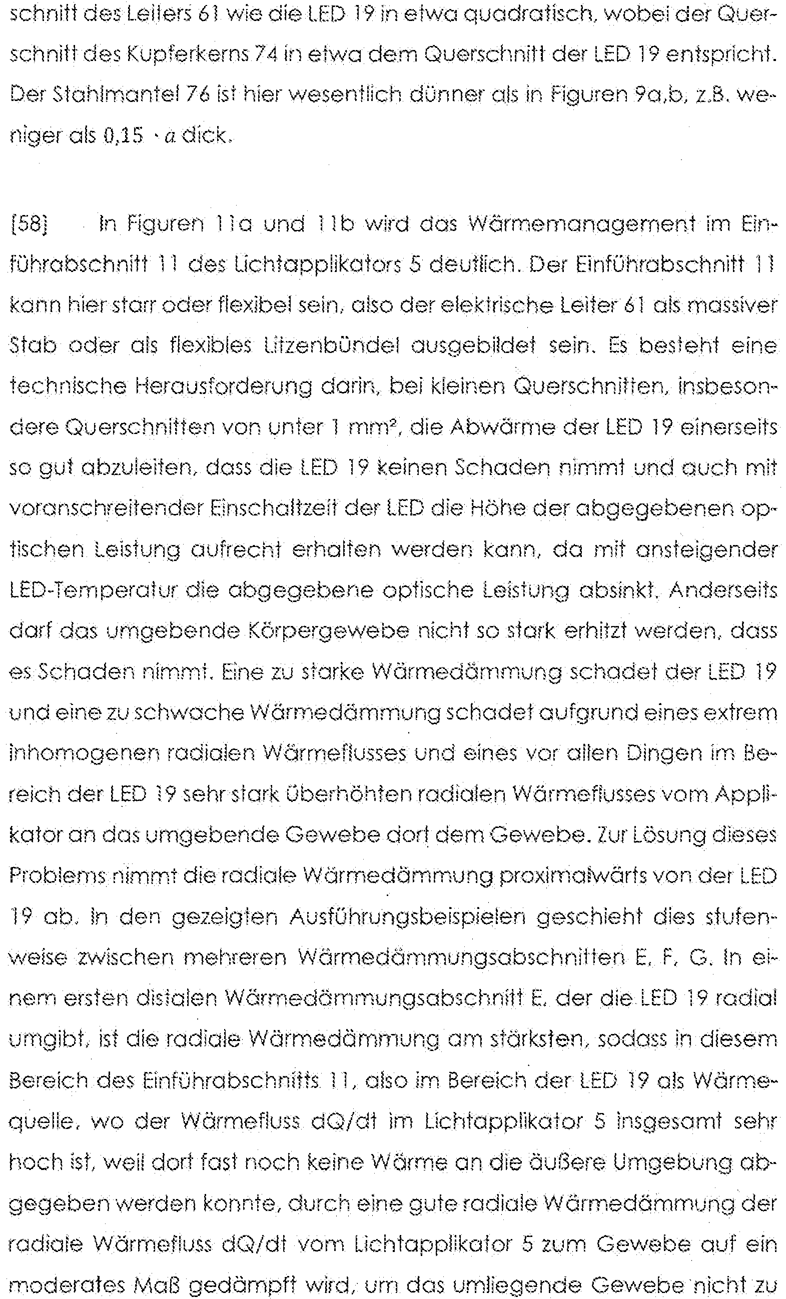

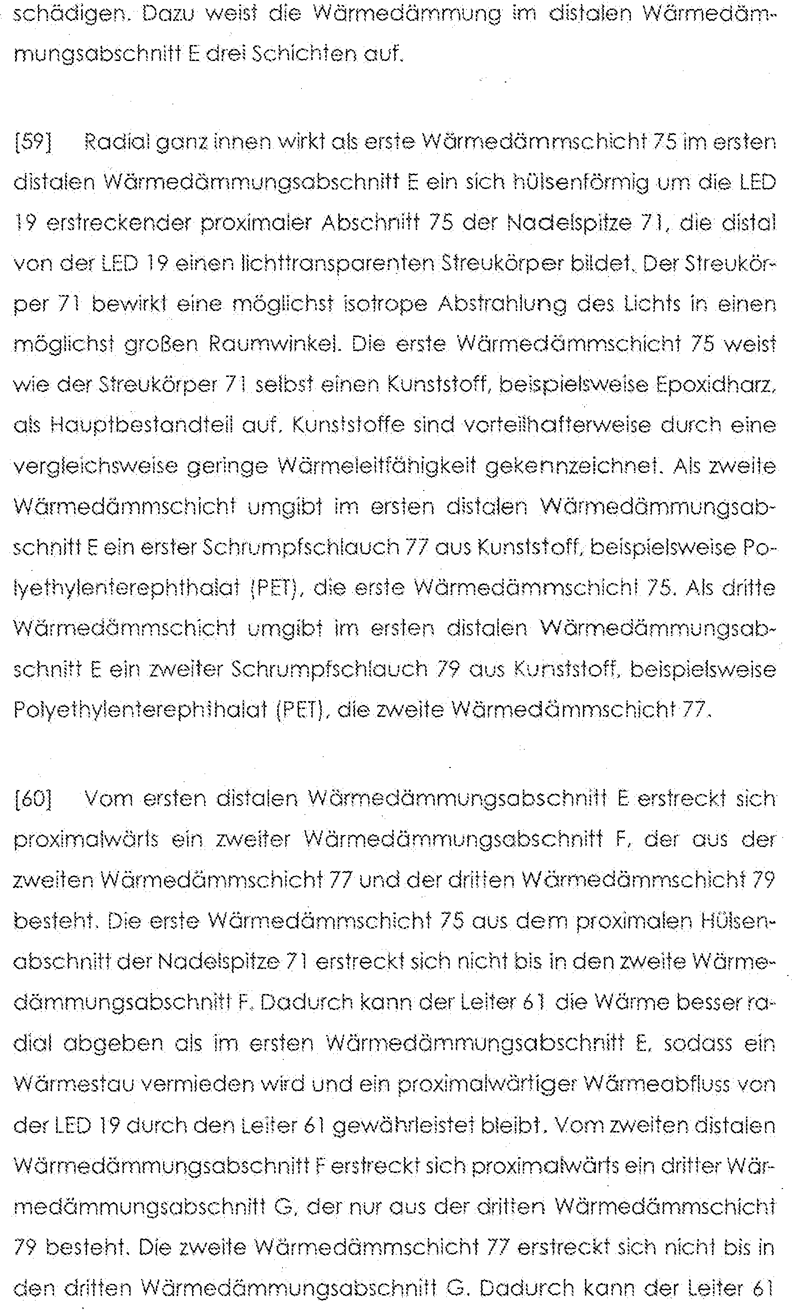

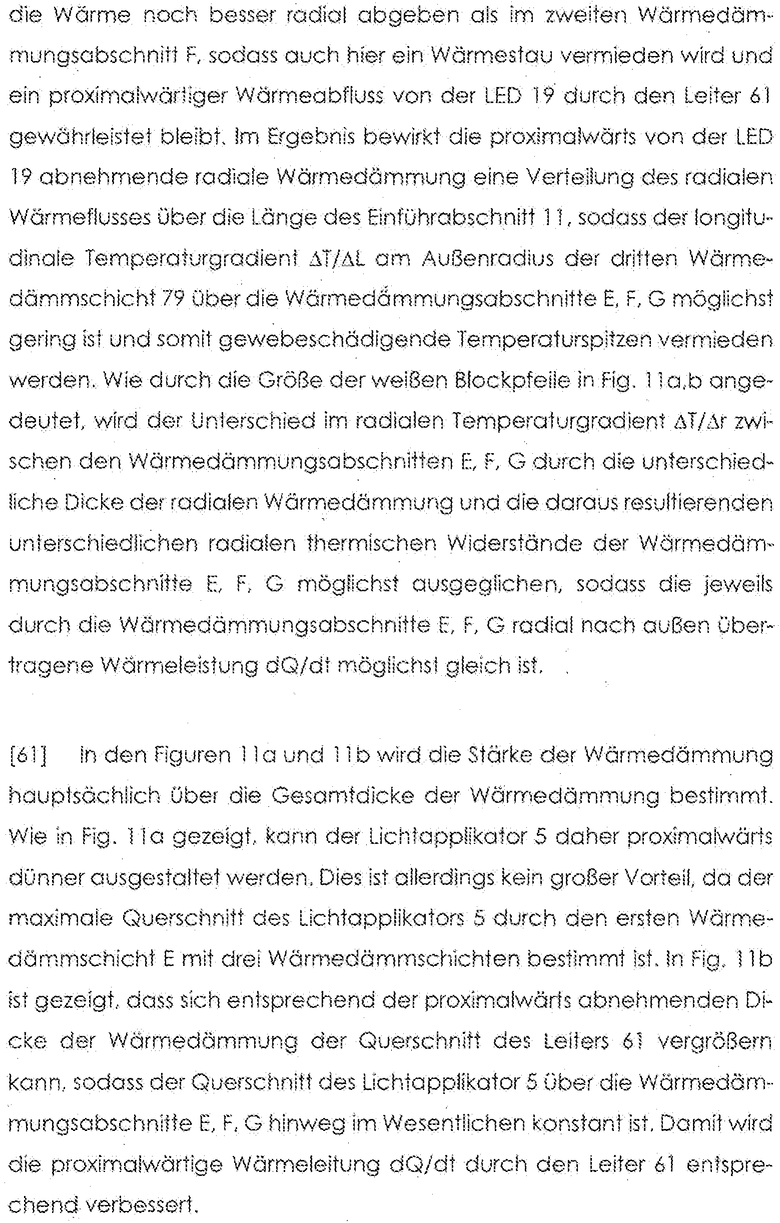

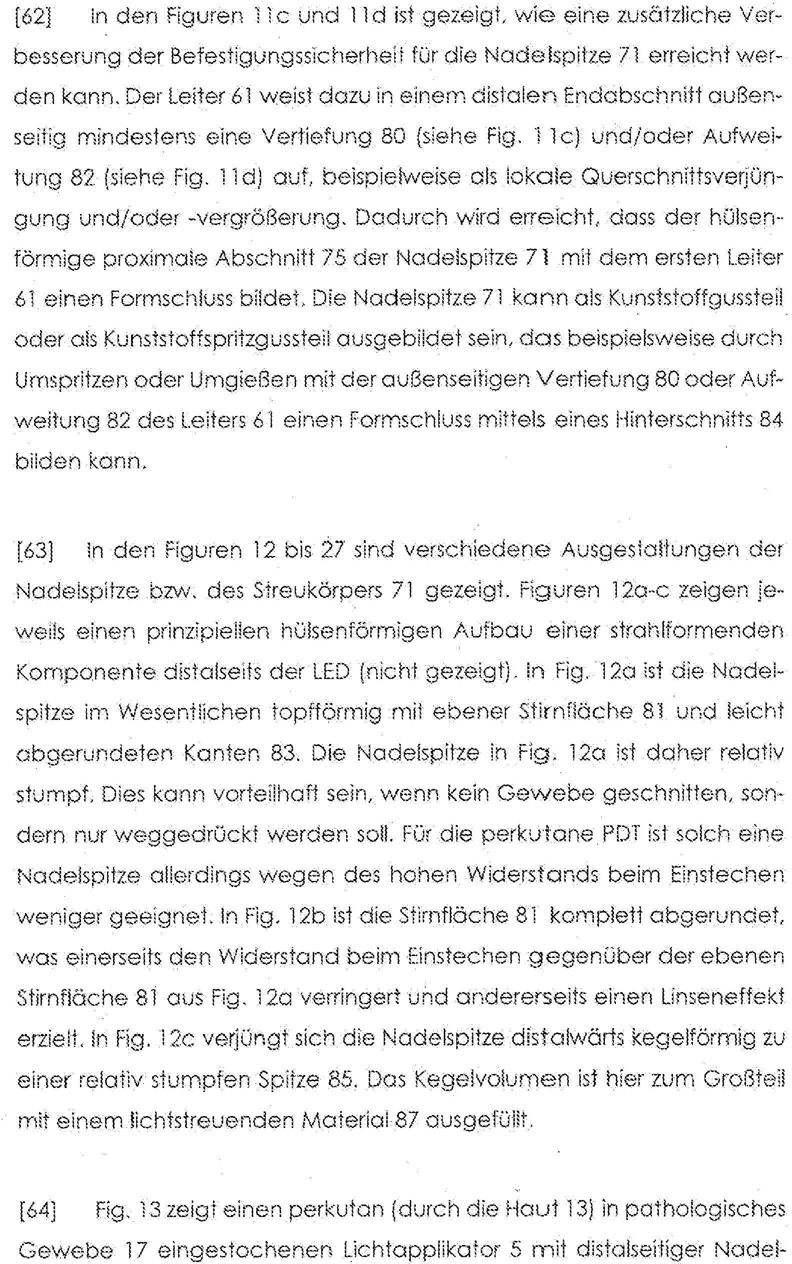

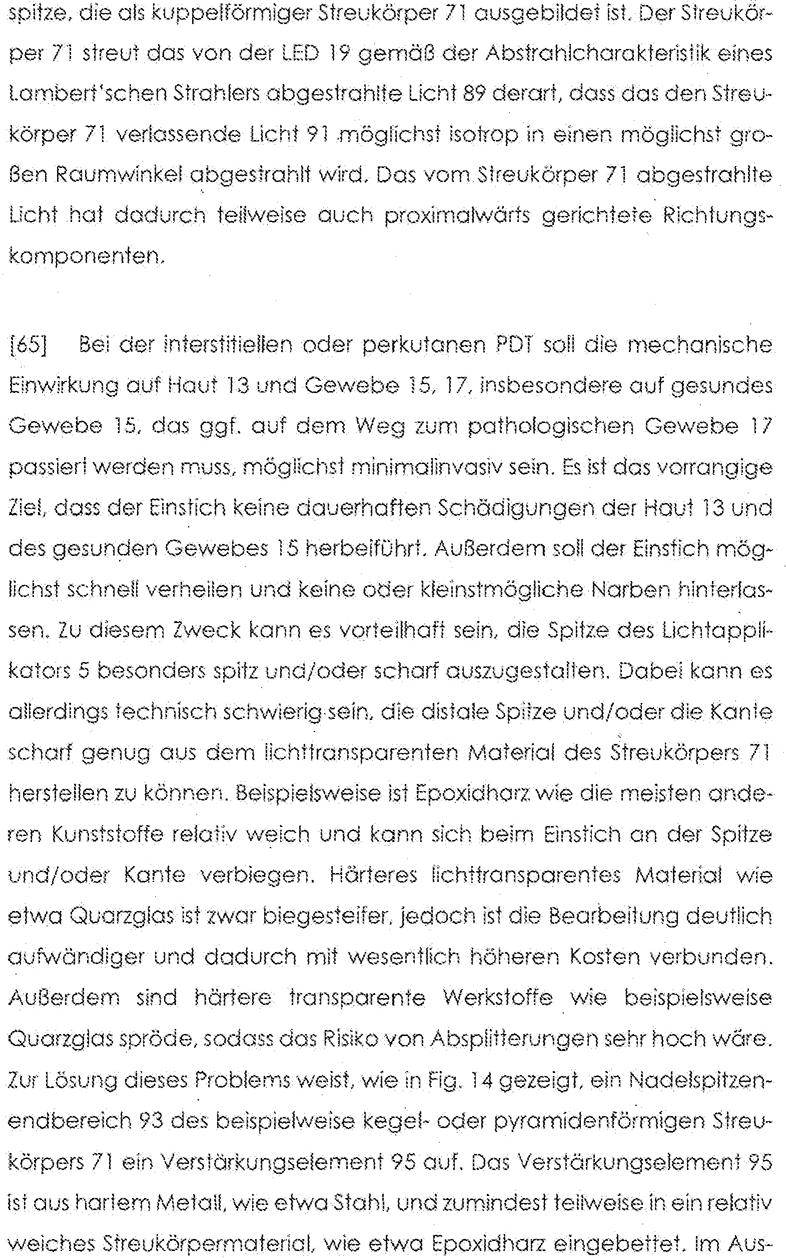

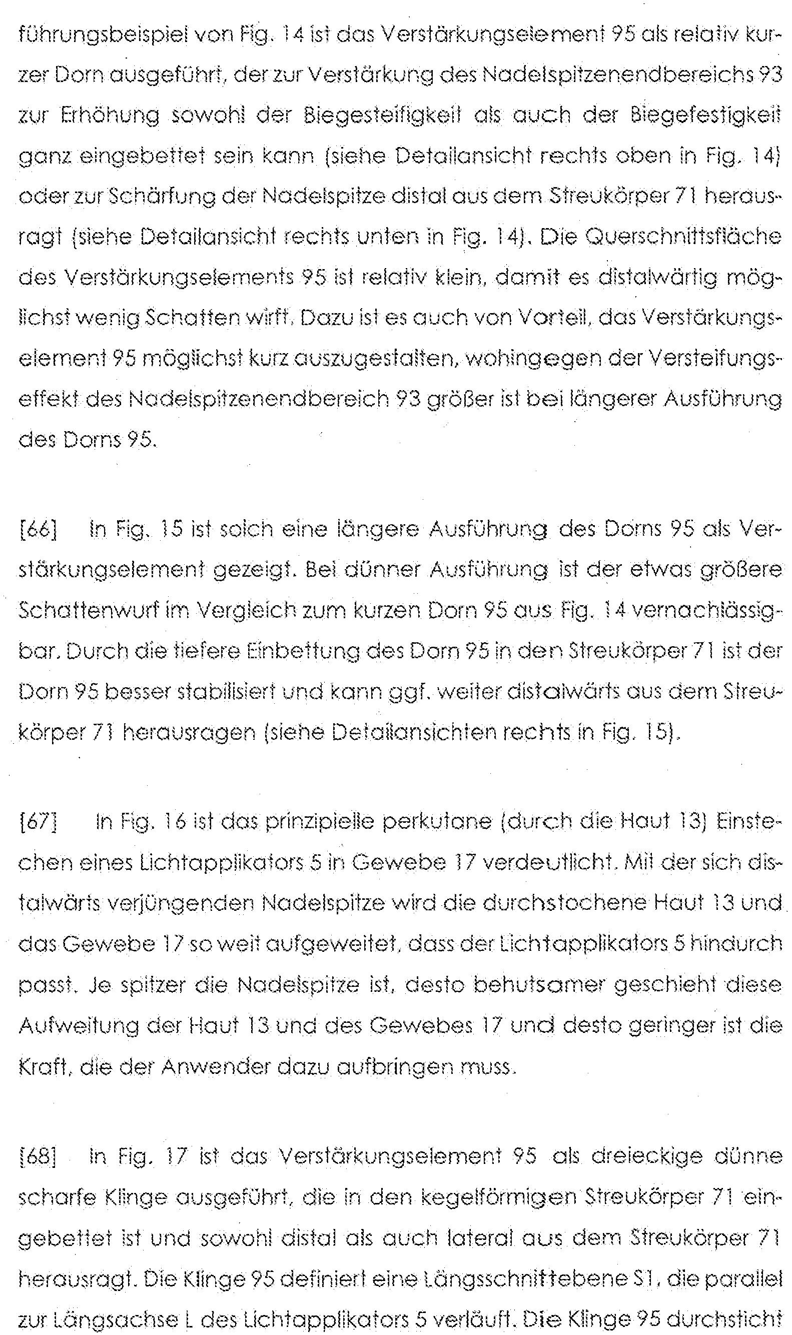

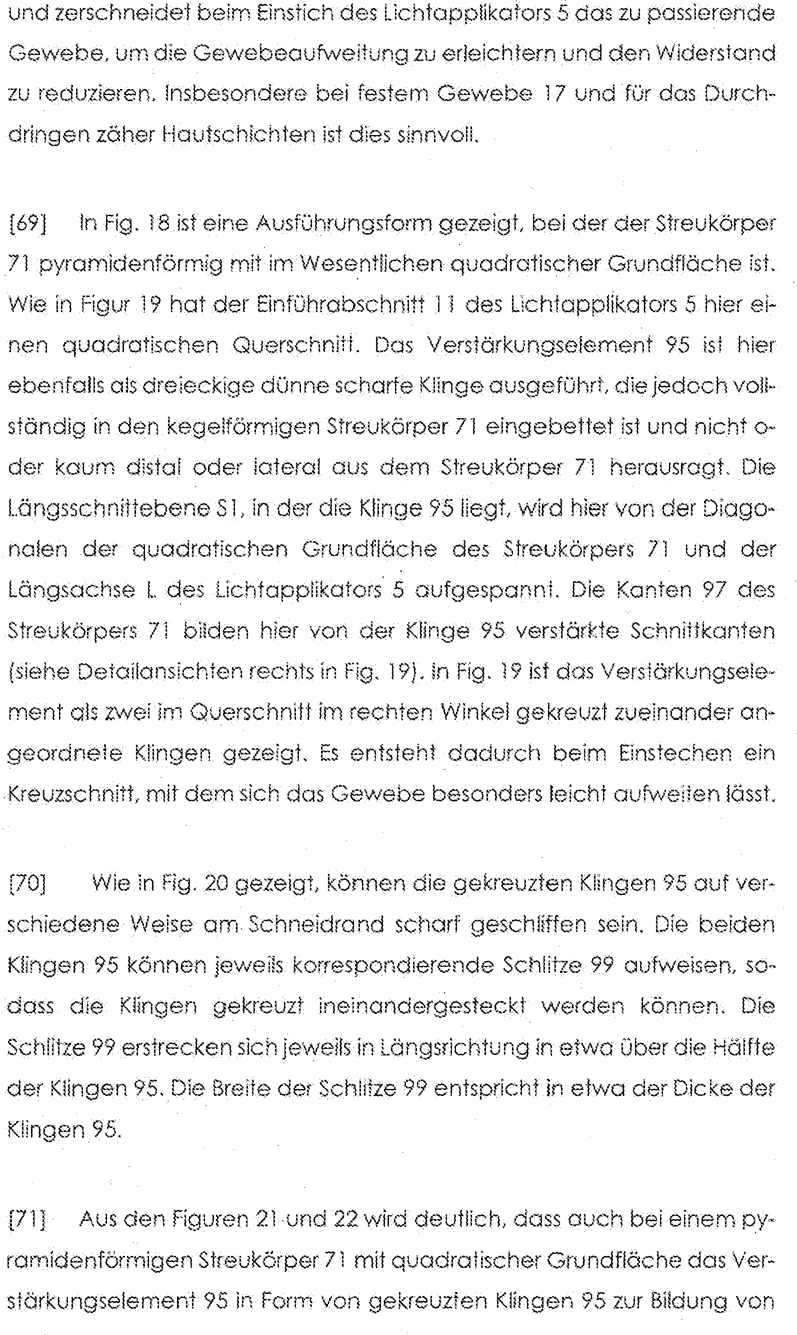

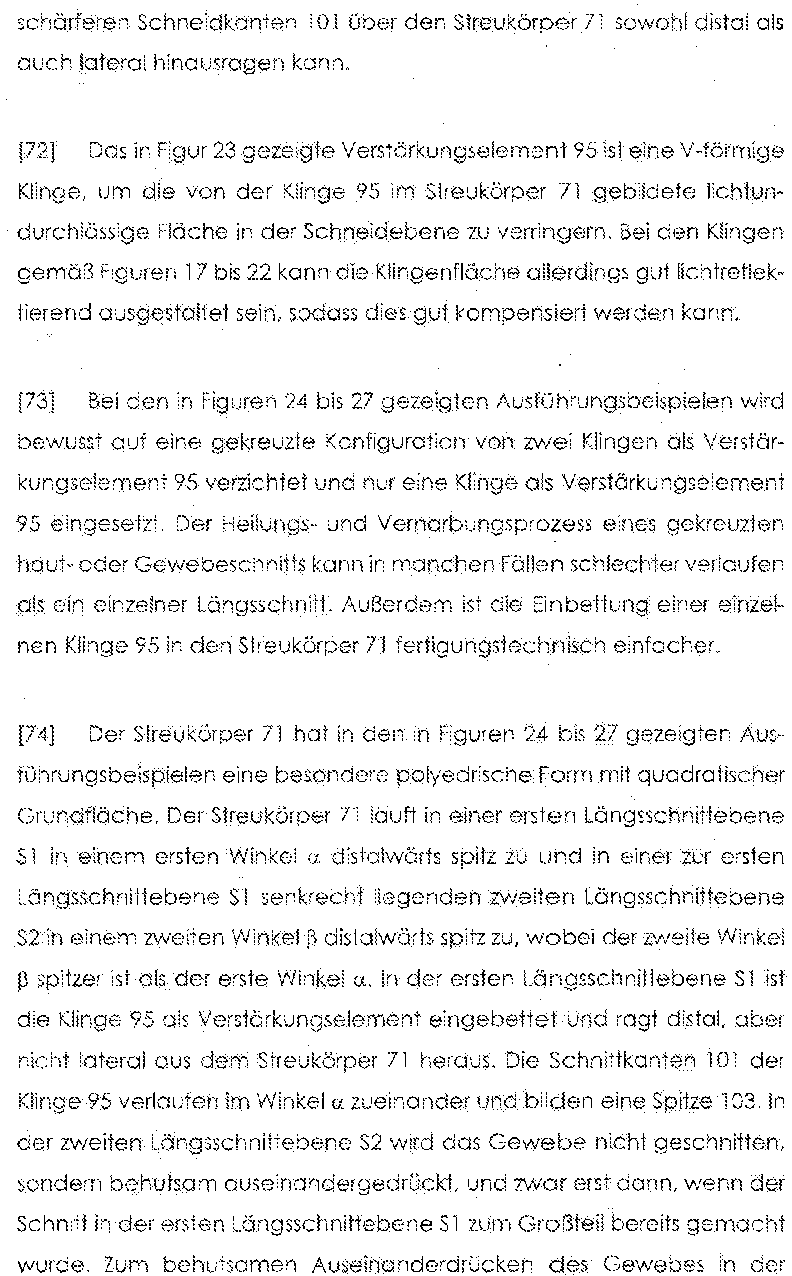

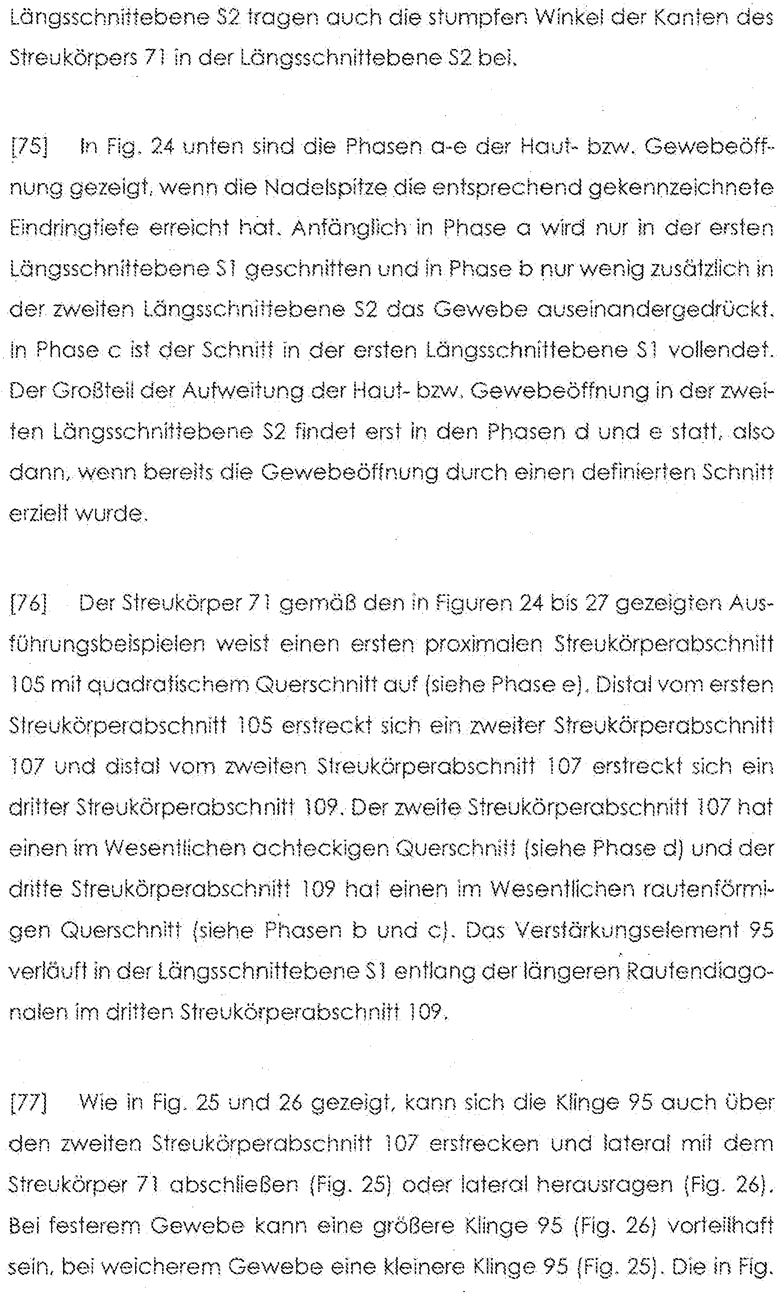

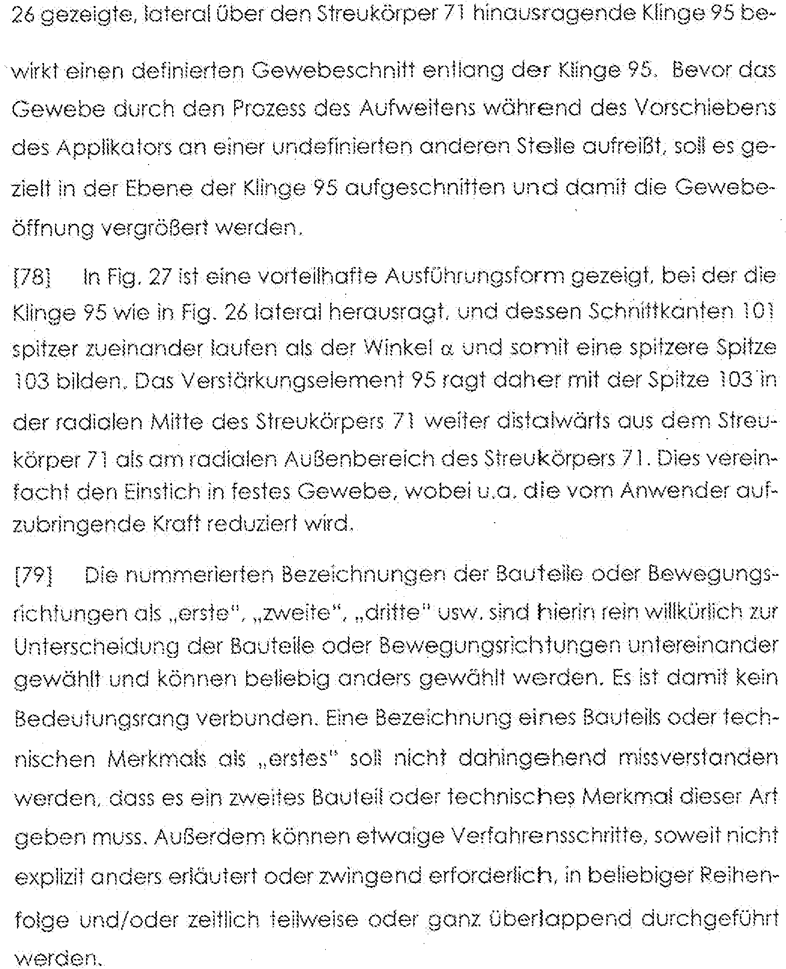

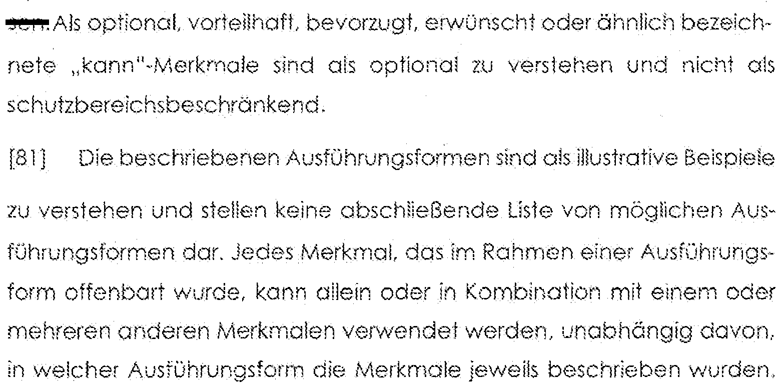

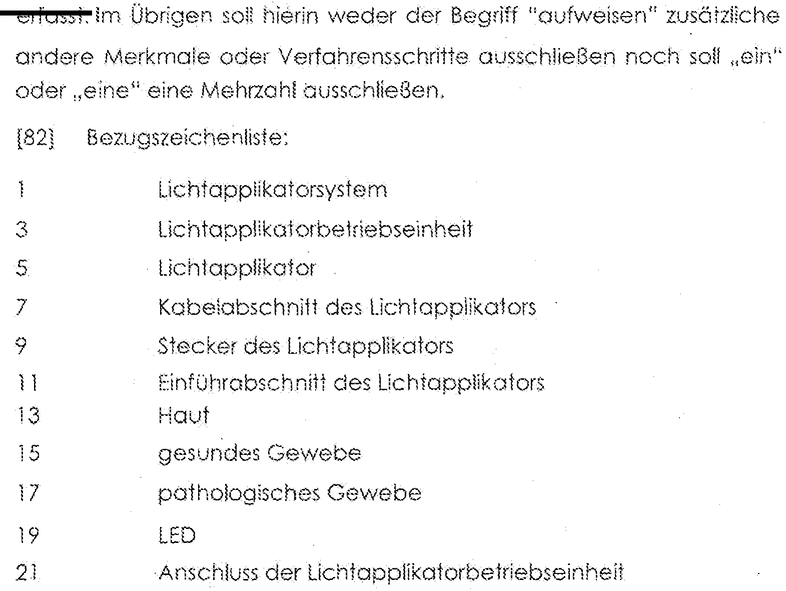

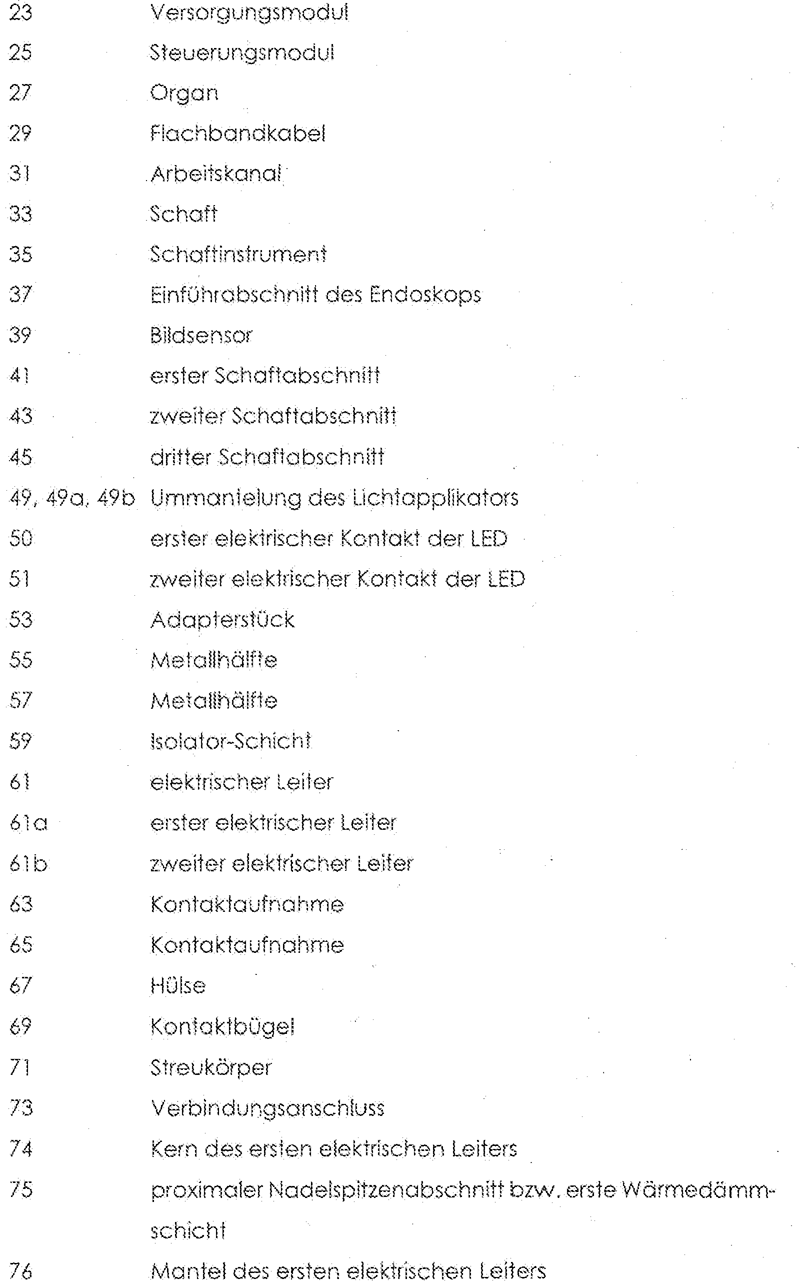

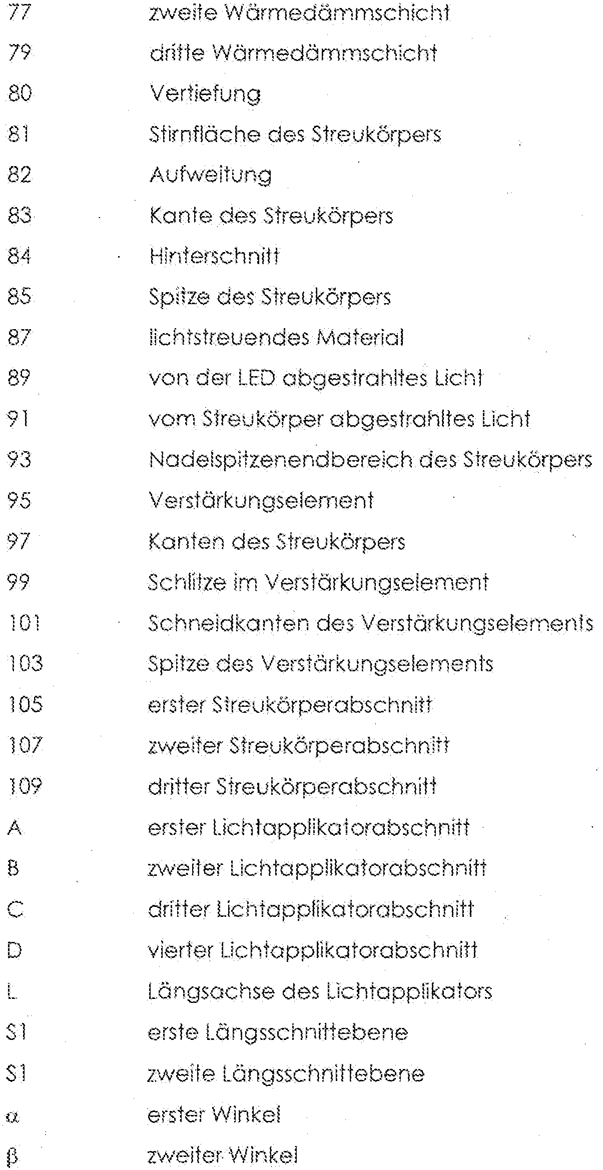

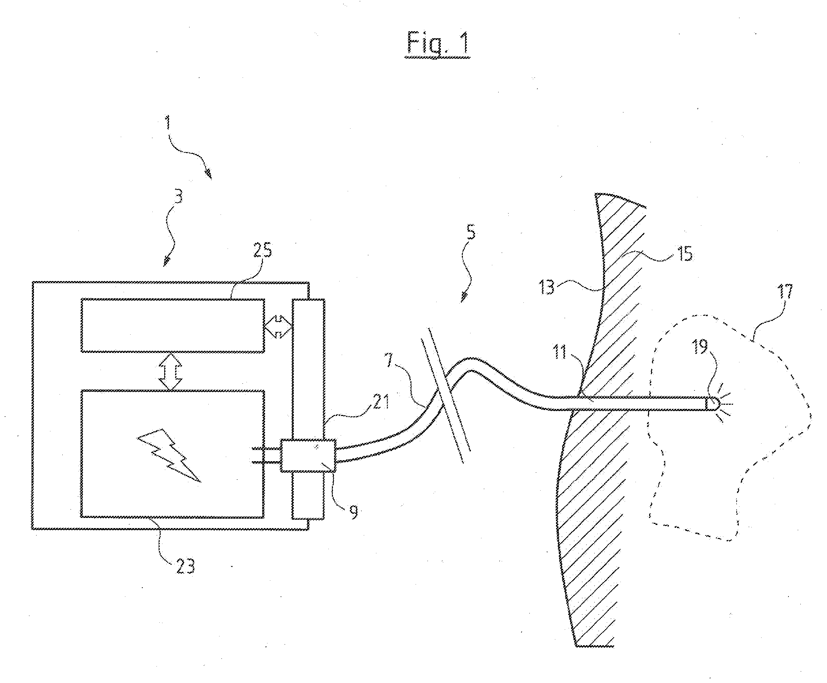

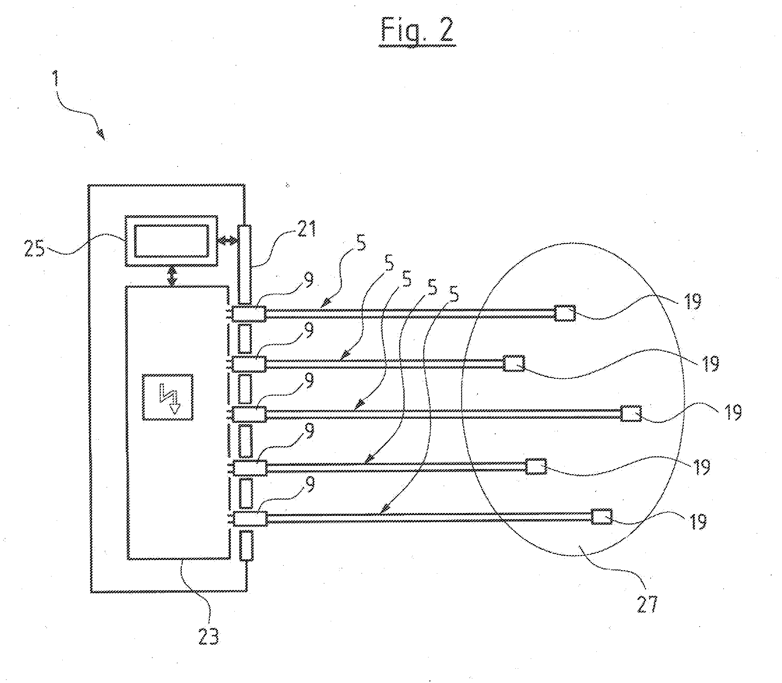

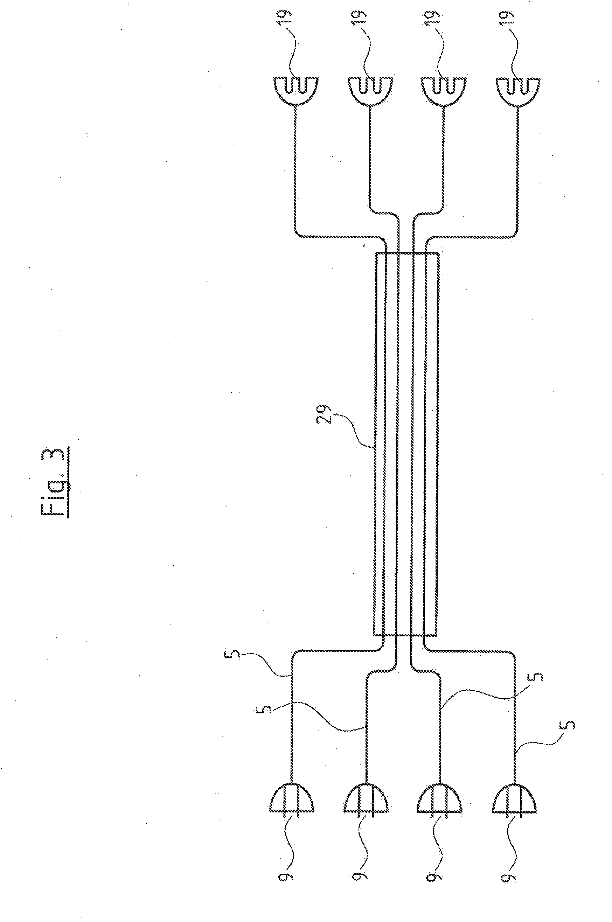

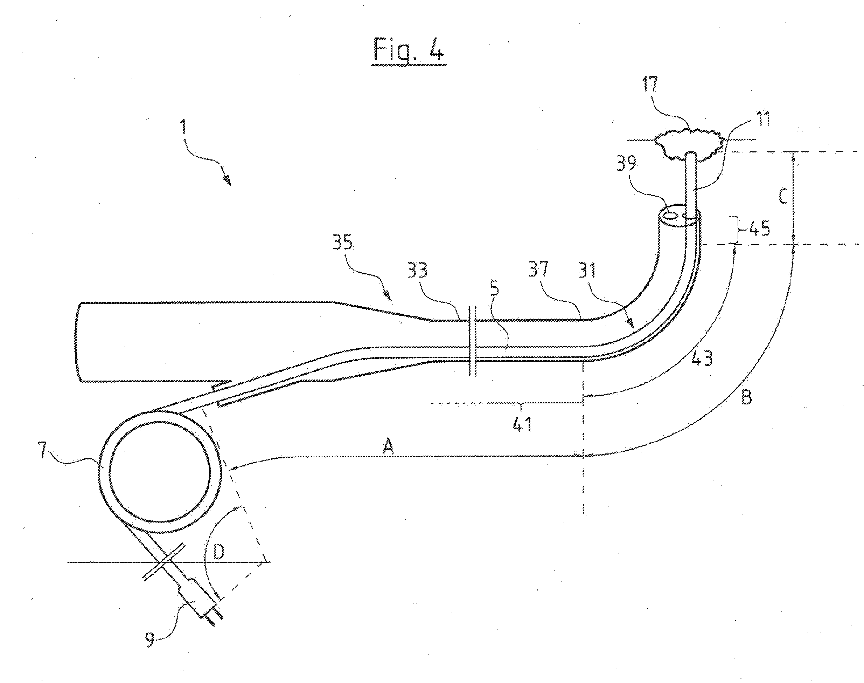

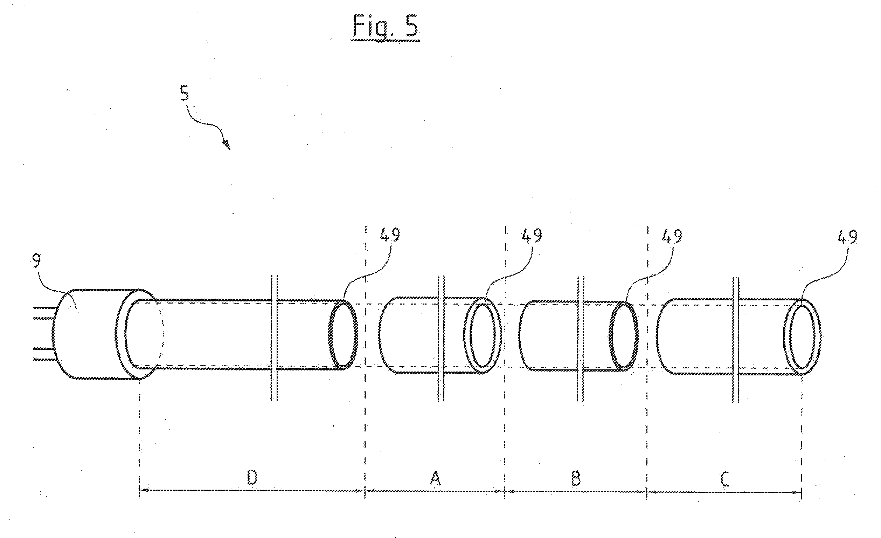

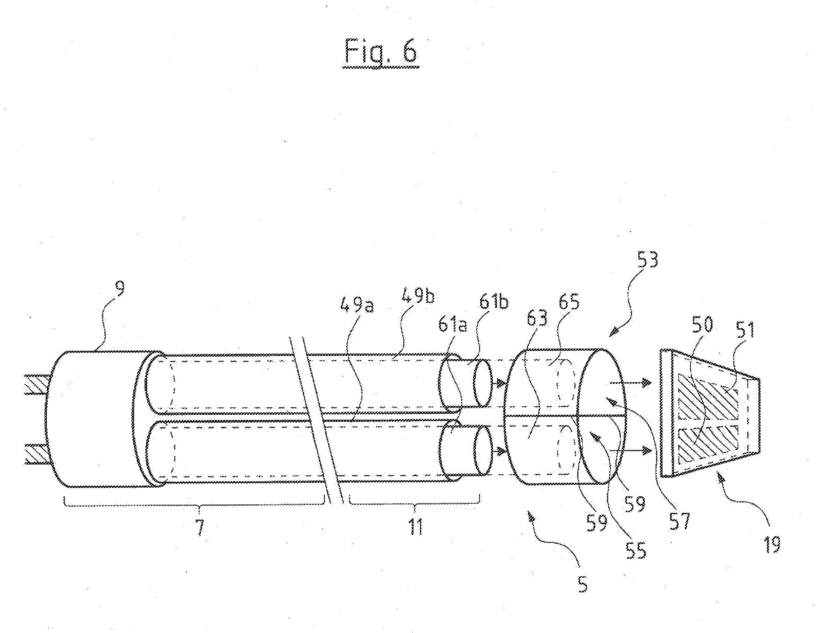

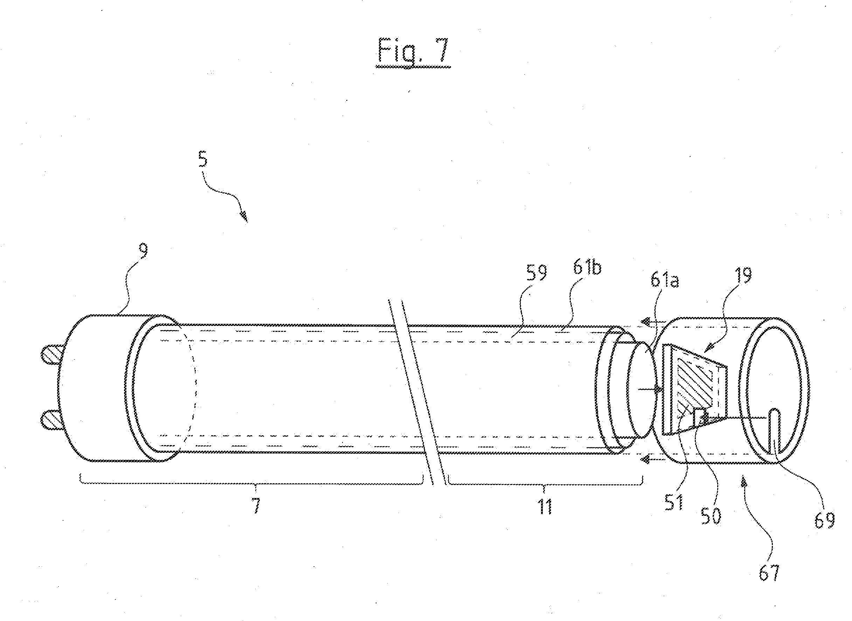

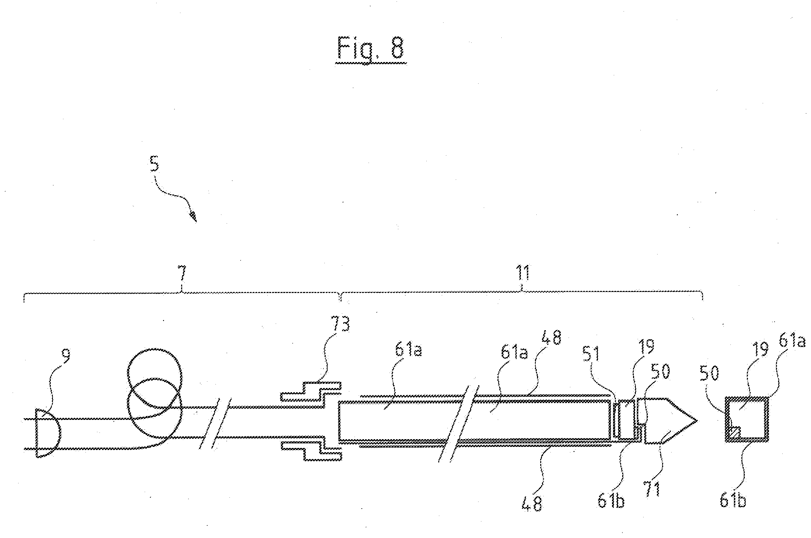

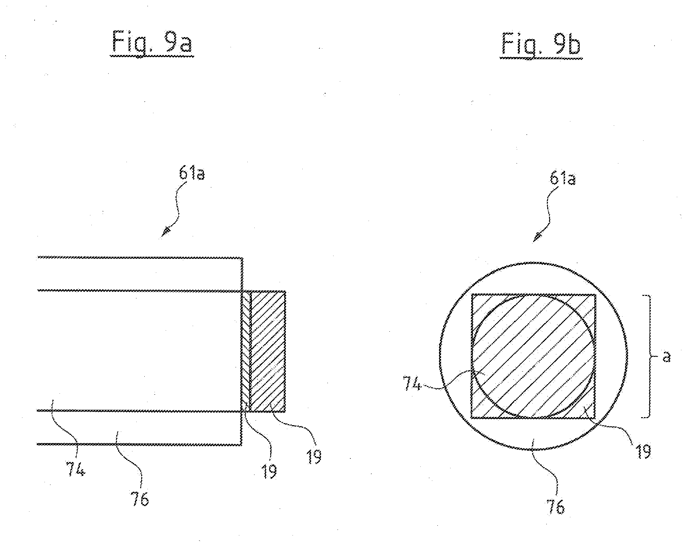

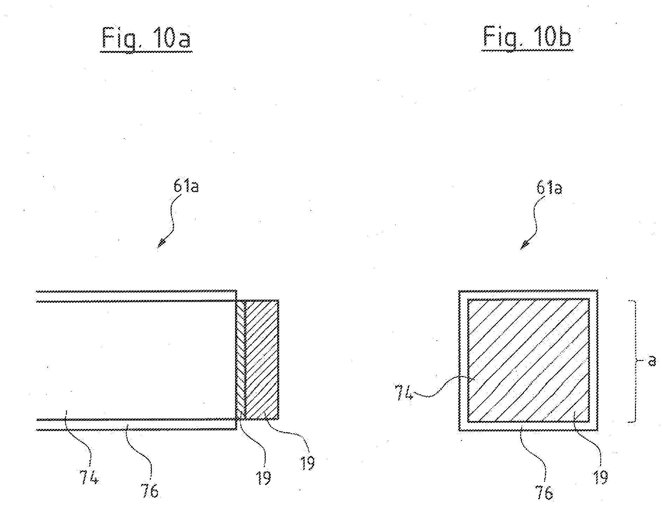

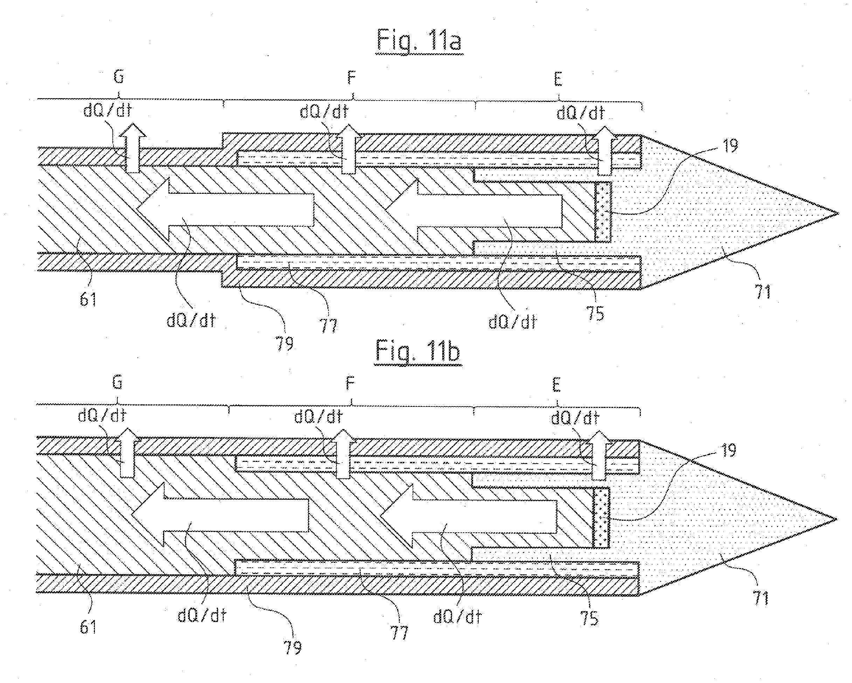

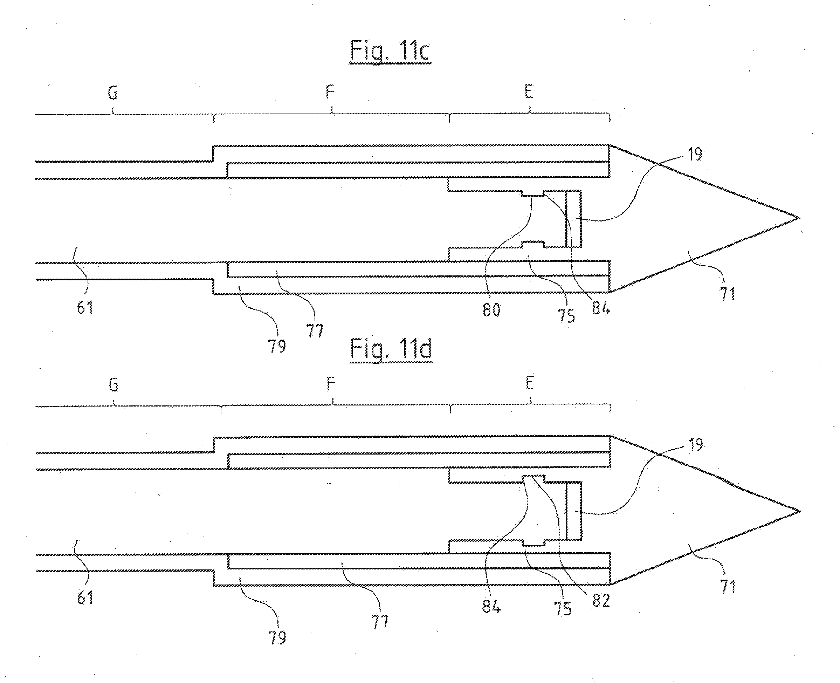

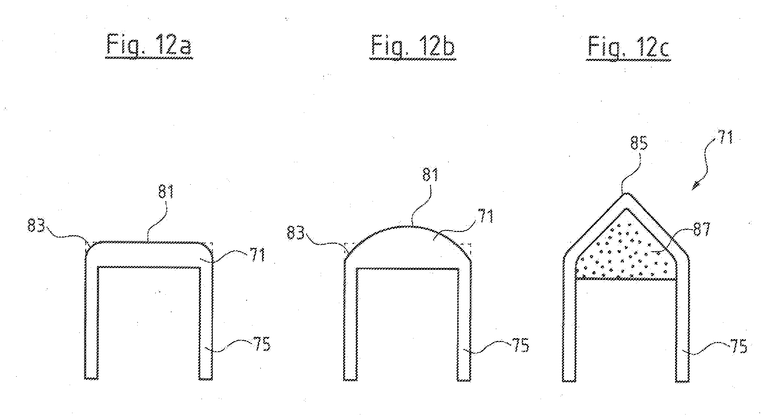

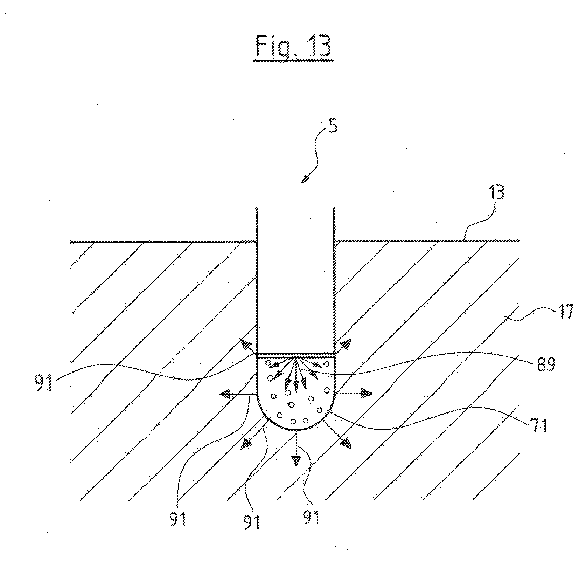

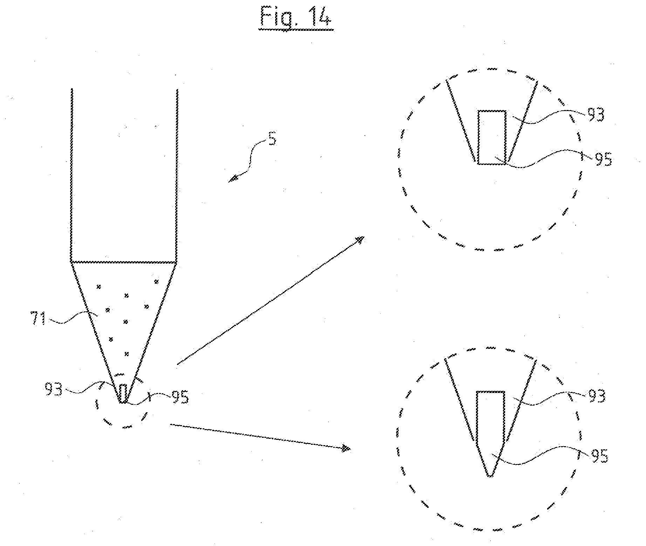

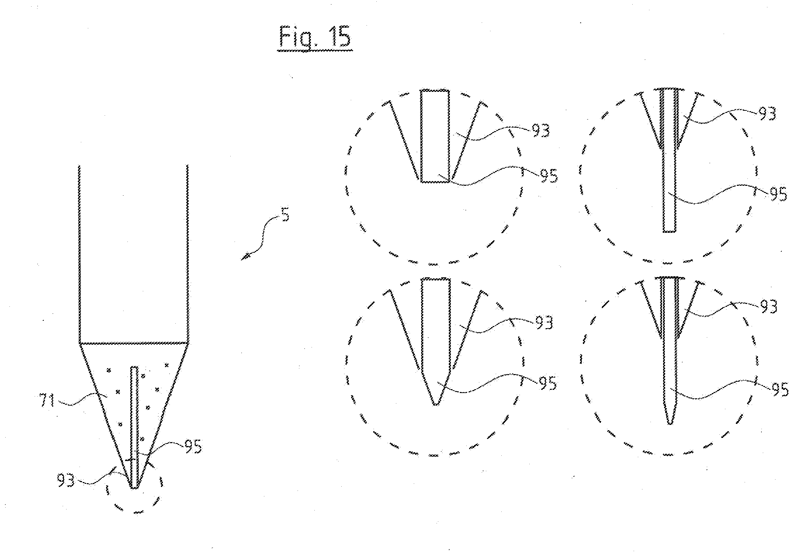

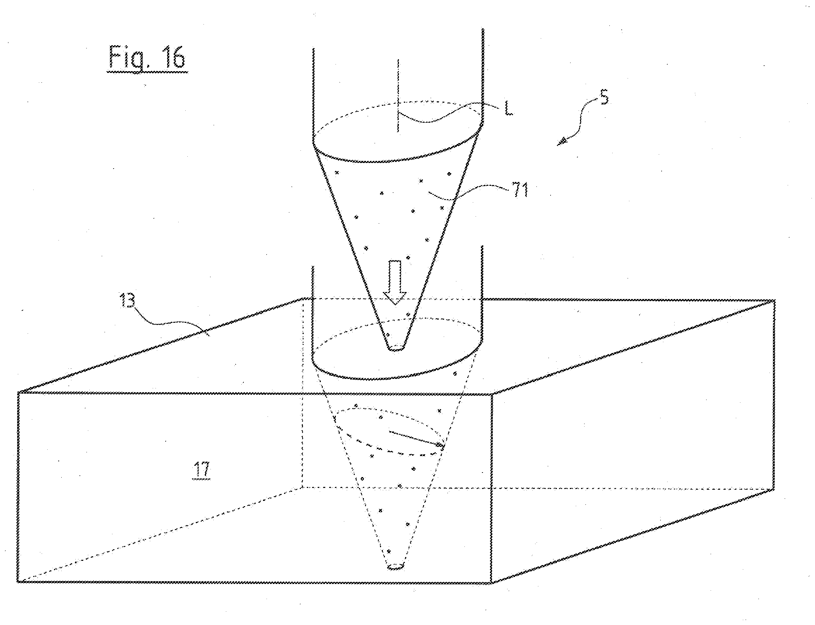

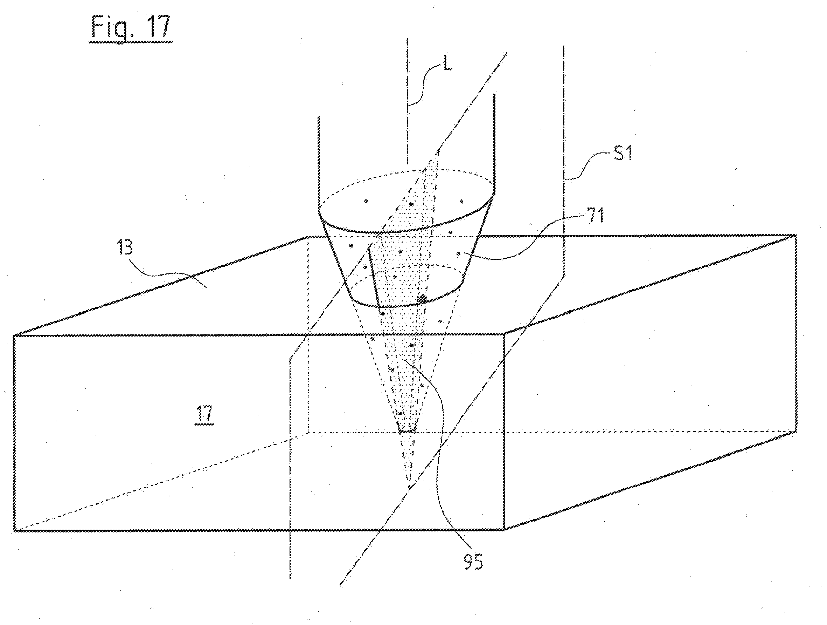

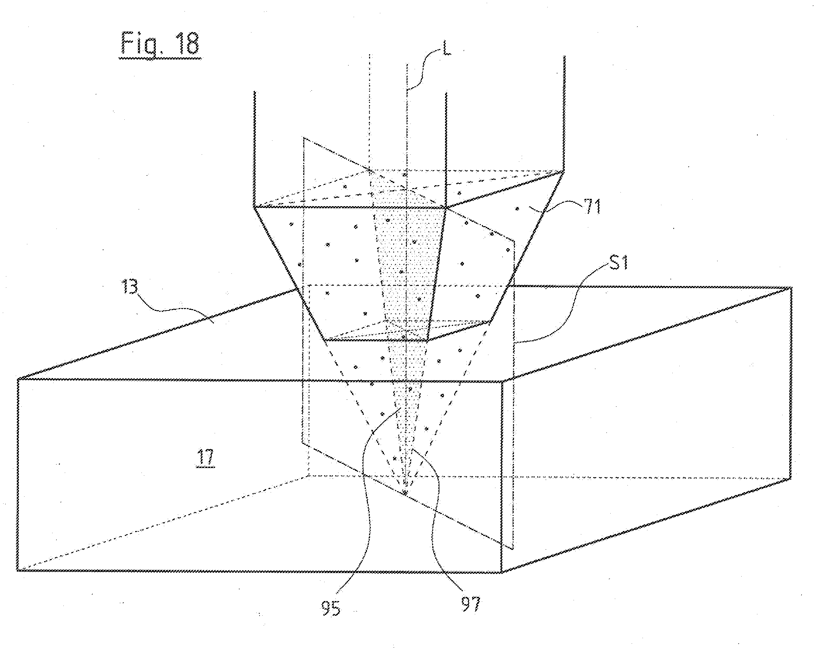

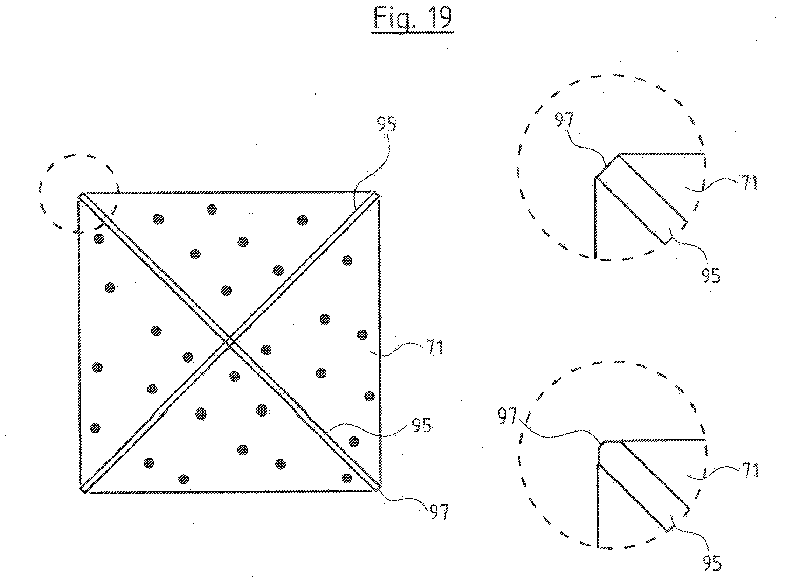

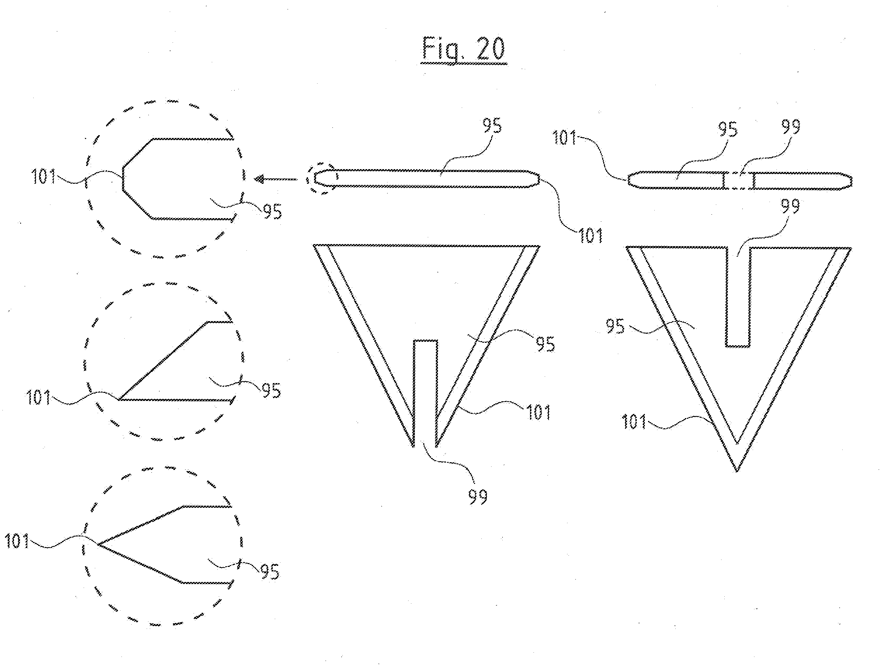

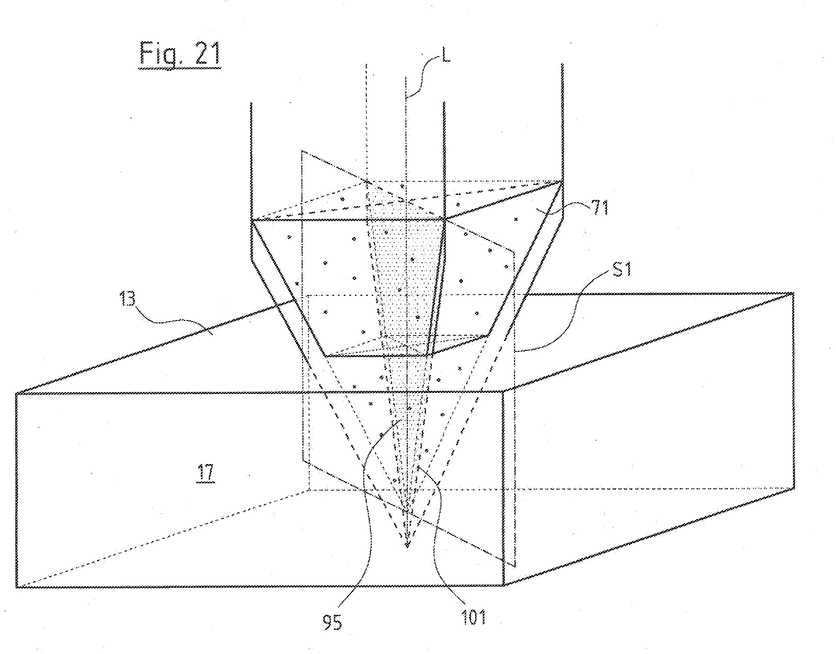

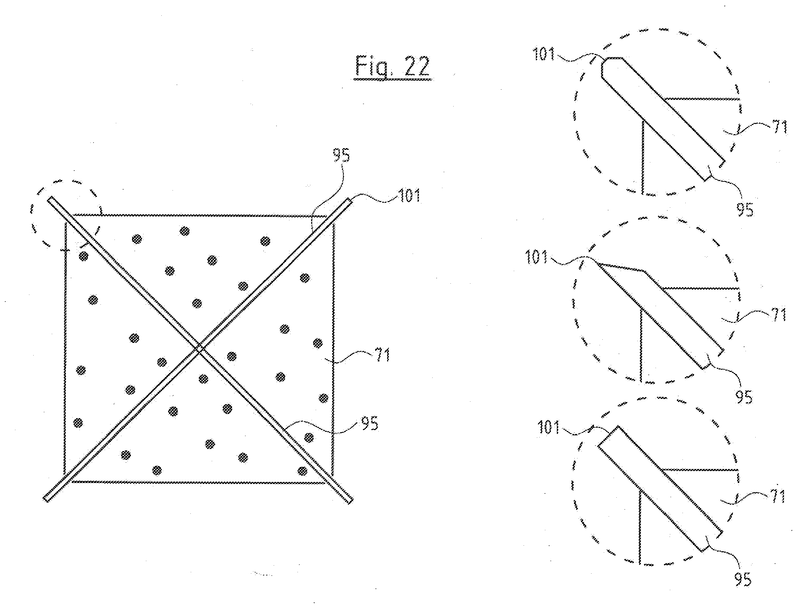

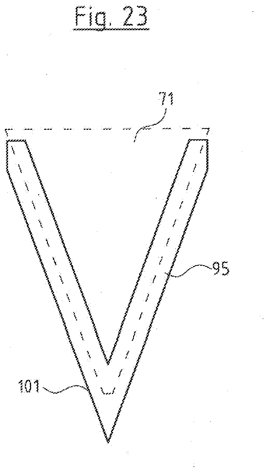

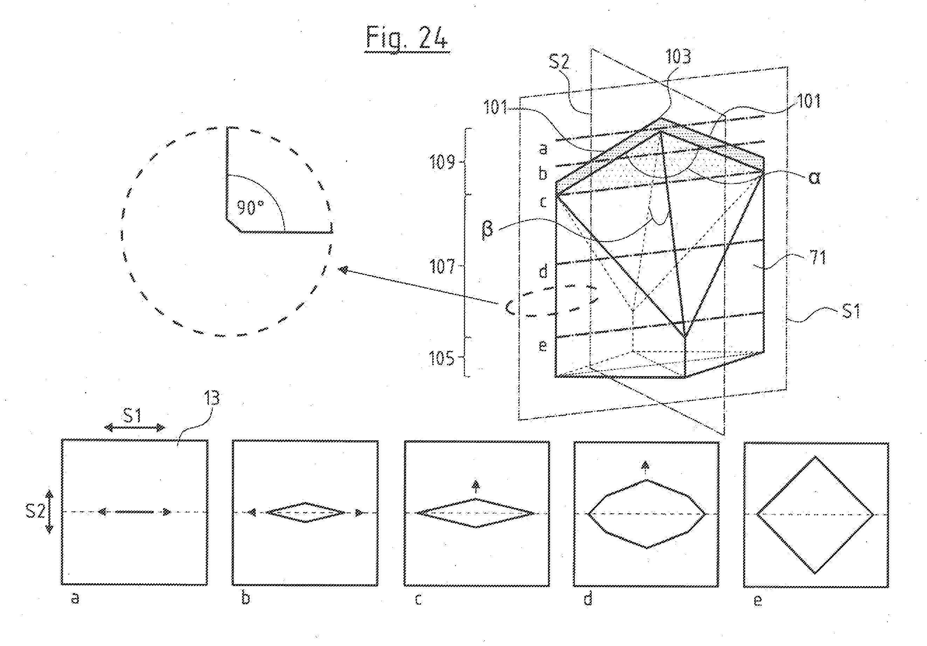

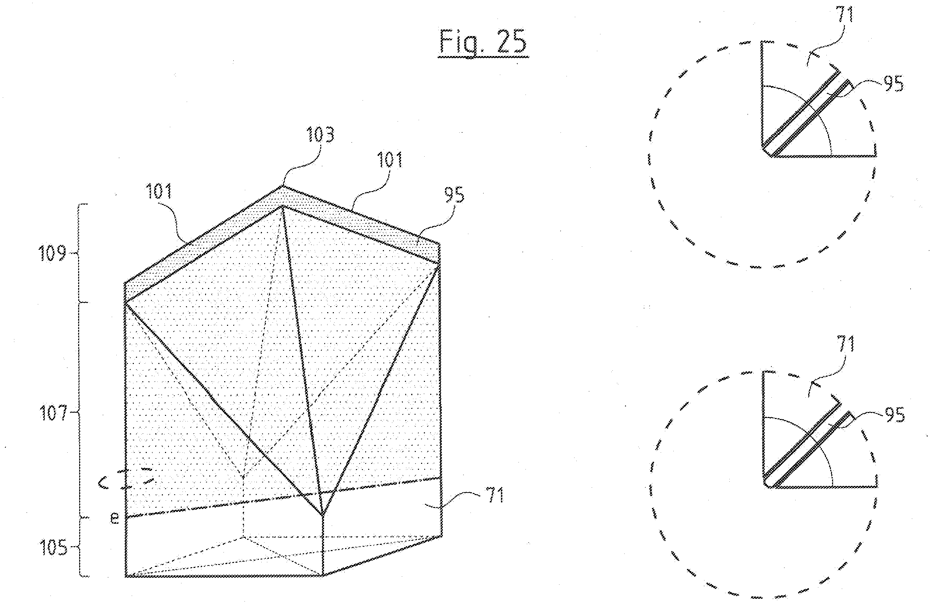

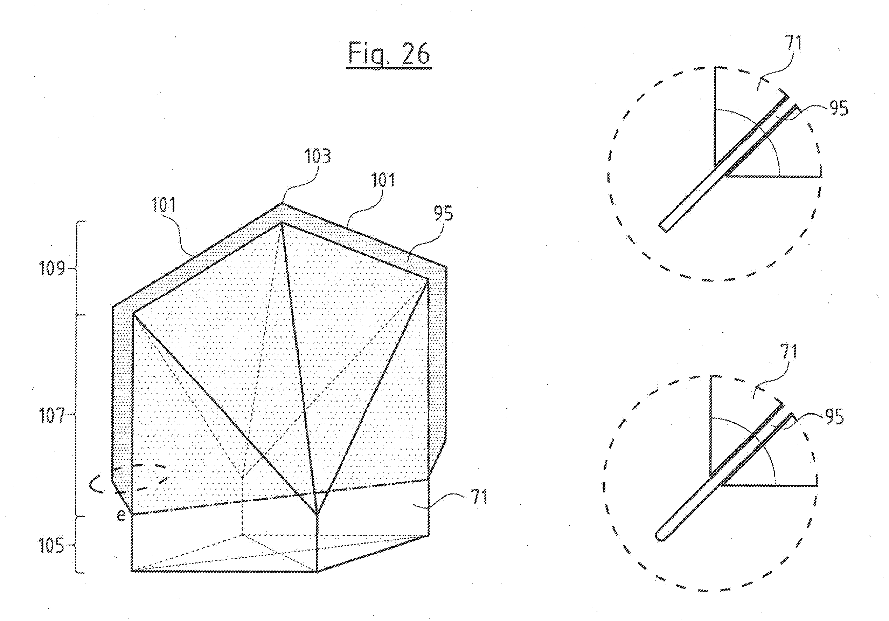

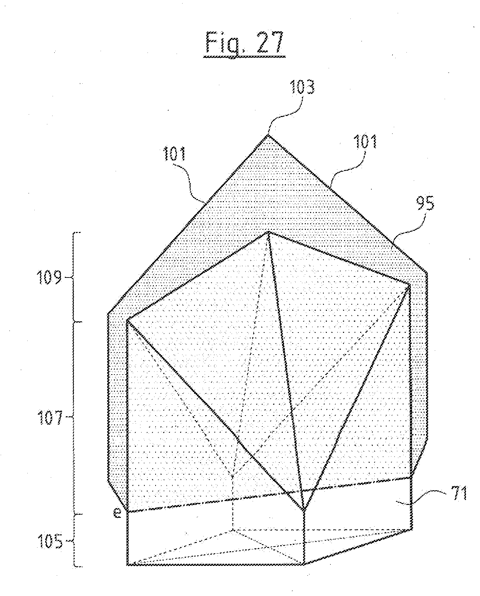

## Patentansprüche

1. Lichtapplikator (5) zur Untersuchung und/oder Behandlung von einem organischen Körper, wobei der Lichtapplikator (5) einen minimalinvasiven starren, semi-flexiblen oder flexiblen Einführabschnitt (11) aufweist, der sich entlang einer Längsrichtung (L) erstreckt und an seinem distalen Ende eine LED (19) aufweist, wobei der Lichtapplikator (5) einen ersten elektrischen Leiter (61a) zur Stromversorgung der LED (19) aufweist, der sich im Einführabschnitt (11) in Längsrichtung (L) erstreckt und dort eine Querschnittsfläche von mindestens 70% der Querschnittsfläche des Lichtapplikators (5) hat, wobei der Lichtapplikator (5) im Einführabschnitt (11) in radialer Richtung wärmegedämmt ist, **dadurch gekennzeichnet, dass** der Lichtapplikator (5) im Einführabschnitt (11) in radialer Richtung derart wärmegedämmt ist, dass die radiale Wärmedämmung proximalwärts abnimmt.

2. Lichtapplikator (5) nach Anspruch 1, wobei die radiale Wärmedämmung im Einführabschnitt (11) proximalwärts stufenweise und/oder stetig abnimmt.

3. Lichtapplikator (5) nach Anspruch 1 oder 2, wobei der Lichtapplikator (5) im Einführabschnitt (11) mindestens eine radiale Wärmeisolationsschicht (75, 77, 79) aufweist, wobei die Gesamtdicke der mindestens einen Wärmeisolationsschicht (75, 77, 79) proximalwärts abnimmt.

4. Lichtapplikator (5) nach einem der vorhergehenden Ansprüche, wobei der Lichtapplikator (5) im Einführabschnitt (11) eine Mehrzahl von radialen Wärmeisolationsschichten (75, 77, 79) aufweist, wobei die Anzahl der Wärmeisolationsschichten (75, 77, 79) proximalwärts abnimmt.

5. Lichtapplikator (5) nach einem der vorhergehenden Ansprüche, wobei der Durchmesser des ersten Leiters (61a) in dem Maße zunimmt wie die radiale Wärmedämmung proximalwärts abnimmt.

6. Lichtapplikator (5) nach einem der vorhergehenden Ansprüche, wobei der Lichtapplikator (5) im Einführabschnitt die Wärmeleitfähigkeit des Materials der radialen Wärmedämmung proximalwärts abnimmt.

7. Lichtapplikator (5) nach einem der vorhergehenden Ansprüche, wobei der erste Leiter (61a) außenseitig mindestens eine Vertiefung (80) und/oder Aufweitung (82) aufweist.

8. Lichtapplikator (5) nach einem der vorhergehenden Ansprüche, wobei der Einführabschnitt (11) als starrer Nadelabschnitt ausgebildet ist, wobei der erste Leiter (61a) den Einführabschnitt (11) versteift, und wobei der Lichtapplikator (5) einen proximalseitig mit dem Nadelabschnitt (11) fest verbundenen oder lösbar verbindbaren Kabelabschnitt (7) aufweist.

9. Lichtapplikator (5) nach einem der vorhergehenden Ansprüche, wobei der erste Leiter (61a) einen Kern (74) mit einem ersten Material und einen Mantel (76) mit einem zweiten Material aufweist, wobei das erste Material wärmeleitfähiger ist als das zweite Material und das zweite Material biegesteifer ist als das erste Material.

10. Lichtapplikator (5) nach Anspruch 9, wobei das erste Material Kupfer, Aluminium oder Silber und das zweite Material Stahl aufweist.

11. Lichtapplikator (5) nach Anspruch 9 oder 10, wobei die Querschnittsfläche des Kerns (74) mehr als 40% der Querschnittsfläche des ersten Leiters (61a) beträgt.

12. Lichtapplikator (5) nach einem der Ansprüche 9 bis 11, wobei die Querschnittsfläche des Kerns (74) das 0,8 bis 1,2-fache der Querschnittsfläche der LED (19) beträgt.

13. Lichtapplikator (5) nach einem der Ansprüche 9 bis 12, wobei der der Kern und der Mantel ein Hin- und Rückleiterpaar bilden, wobei der Kern und der Mantel durch eine elektrisch nichtleitende Schicht, die zwischen dem Kern und dem Mantel angeordnet ist, gegeneinander elektrisch isoliert sind.

14. Lichtapplikator (5) nach einem der vorhergehenden Ansprüche, wobei die Querschnittsfläche des ersten Leiters (61a) mindestens so groß wie die Querschnittsfläche der LED (19) ist.

15. Lichtapplikator (5) nach einem der vorhergehenden Ansprüche, wobei die LED (19) in elektrischem und wärmeleitendem Kontakt mit einer distalen Stirnfläche des ersten Leiters (61a) steht.

## Claims

1. Light-delivery unit (5) for the examination and/or treatment of an organic body, wherein the light-delivery unit (5) has a minimally invasive rigid, semi-flexible or flexible, insertion section (11), which extends along a longitudinal direction (L) and at its distal end has an LED (19), wherein the light-delivery unit (5) has a first electrical conductor (61a) for the supply of power to the LED (19), which conductor extends in the insertion section (11) in the longitudinal direction (L), and there has a cross-sectional area of at least 70% of the cross-sectional area of the light-delivery unit (5), wherein in the insertion section (11) the light-delivery unit (5) is thermally insulated in the radial direction, **characterised in that**, in the insertion section (11) the light-delivery unit (5) is thermally insulated in the radial direction in such a way that the radial thermal insulation decreases in the proximal direction.

2. Light-delivery unit (5) according to Claim 1, wherein in the insertion section (11) the radial thermal insulation decreases in steps, and/or steadily, in the proximal direction.

3. Light-delivery unit (5) according to Claim 1 or 2, wherein in the insertion section (11) the light-delivery unit (5) has at least one radial thermal insulation layer (75, 77, 79), wherein the total thickness of the at least one thermal insulation layer (75, 77, 79) decreases in the proximal direction.

4. Light-delivery unit (5) according to one of the preceding claims, wherein in the insertion section (11) the light-delivery unit (5) has a number of radial thermal insulation layers (75, 77, 79), wherein the number of thermal insulation layers (75, 77, 79) decreases in the proximal direction.

5. Light-delivery unit (5) according to one of the preceding claims, wherein the diameter of the first conductor (61a) increases, as the radial thermal insulation decreases, in the proximal direction.

6. Light-delivery unit (5) according to one of the preceding claims, wherein in the insertion section of the light-delivery unit (5), the thermal conductivity of the material of the radial thermal insulation decreases in the proximal direction.

7. Light-delivery unit (5) according to one of the preceding claims, wherein the first conductor (61a) has at least one recess (80), and/or enlargement (82), on its external surface.

8. Light-delivery unit (5) according to one of the preceding claims, wherein the insertion section (11) is designed as a rigid needle section, wherein the first conductor (61a) stiffens the insertion section (11), and wherein on the proximal side the light-delivery unit (5) has a cable section (7), which is fixedly connected, or can be releasably connected, to the needle section (11).

9. Light-delivery unit (5) according to one of the preceding claims, wherein the first conductor (61a) has a core (74) of a first material, and a sheath (76) of a second material, wherein the first material is more thermally conductive than the second material, and the second material is more flexurally rigid than the first material.

10. Light-delivery unit (5) according to Claim 9, wherein the first material comprises copper, aluminium or silver, and the second material comprises steel.

11. Light-delivery unit (5) according to Claim 9 or 10, wherein the cross-sectional area of the core (74) is more than 40% of the cross-sectional area of the first conductor (61a).

12. Light-delivery unit (5) according to one of the Claims 9 to 11, wherein the cross-sectional area of the core (74) is 0.8 to 1.2 times the cross-sectional area of the LED (19).

13. Light-delivery unit (5) according to one of the Claims 9 to 12, wherein the core and the sheath form a forward and return conductor pair, wherein the core and the sheath are electrically insulated from each other by an electrically non-conducting layer, which is arranged between the core and the sheath.

14. Light-delivery unit (5) according to one of the preceding claims, wherein the cross-sectional area of the first conductor (61a) is at least as large as the cross-sectional area of the LED (19).

15. Light-delivery unit (5) according to one of the preceding claims, wherein the LED (19) is in electrical and heat-conducting contact with a distal end face of the first conductor (61a).

## Revendications

1. Applicateur de distribution de lumière (5) pour examiner et/ou traiter un corps organique, l'applicateur de distribution de lumière (5) comprenant une partie d'introduction (11) rigide, semi-flexible ou flexible à invasion minimale qui s'étend le long d'une direction longitudinale (L) et comprend, à son extrémité distale, une DEL (19), l'applicateur de distribution de lumière (5) comprenant un premier conducteur électrique (61a) pour l'alimentation de la DEL (19) qui s'étend dans la partie d'introduction (11) dans la direction longitudinale (L) et qui y a une section transversale d'au moins 70% de la section transversale de l'applicateur de distribution de lumière (5), l'applicateur de distribution de lumière (5) étant isolé thermiquement dans la direction radiale dans la partie d'introduction (11), **caractérisé en ce que** l'applicateur de distribution de lumière (5) est isolé thermiquement dans la direction radiale dans la partie d'introduction (11) de façon telle que l'isolation thermique radiale décroît en direction proximale.

2. Applicateur de distribution de lumière (5) selon la revendication 1, l'isolation thermique radiale dans la partie d'introduction (11) décroissant en direction proximale par étape et/ou de façon continue.

3. Applicateur de distribution de lumière (5) selon l'une des revendications précédentes, l'applicateur de distribution de lumière (5) comprenant dans la partie d'introduction (11) au moins une couche d'isolation thermique radiale (75, 77, 79), l'épaisseur totale de la dite couche d'isolation thermique (75, 77, 79) décroissant en direction proximale.

4. Applicateur de distribution de lumière (5) selon l'une des revendications précédentes, l'applicateur de distribution de lumière (5) comprenant dans la partie d'introduction (11) une pluralité de couches d'isolation thermique radiale (75, 77, 79), le nombre de couches d'isolation thermique (75, 77, 79) décroissant en direction proximale.

5. Applicateur de distribution de lumière (5) selon l'une des revendications précédentes, le diamètre du premier conducteur (61a) croissant dans la mesure où l'isolation thermique radiale décroît en direction proximale.

6. Applicateur de distribution de lumière (5) selon l'une des revendications précédentes, **dans la partie d'introduction de l'applicateur de distribution de lumière (5)**, la conductivité thermique du matériau de l'isolation thermique radiale décroissant en direction proximale.

7. Applicateur de distribution de lumière (5) selon l'une des revendications précédentes, le premier conducteur (61a) comprenant du côté extérieur au moins un creux (80) et/ou un élargissement (82).

8. Applicateur de distribution de lumière (5) selon l'une des revendications précédentes, la partie d'introduction (11) étant configurée comme une partie d'aiguille rigide, le premier conducteur (61a) rigidifiant la partie d'introduction (11) et l'applicateur de distribution de lumière (5) comprenant une partie de câble (7) reliée à la partie d'aiguille (11), du côté proximal, de façon constante ou pouvant être connectée de manière amovible.

9. Applicateur de distribution de lumière (5) selon l'une des revendications précédentes, le premier conducteur (61a) comprenant un noyau (74) d'un premier matériau et une enveloppe (76) d'un deuxième matériau, le premier matériau étant davantage conducteur thermique que le deuxième matériau et le deuxième matériau étant davantage rigide en flexion que le premier matériau.

10. Applicateur de distribution de lumière (5) selon la revendication 9, le premier matériau comprenant du cuivre, de l'aluminium ou de l'argent et le deuxième matériau comprenant de l'acier.

11. Applicateur de distribution de lumière (5) selon la revendication 9 ou 10, la section transversale du noyau (74) étant plus que 40% de la section transversale du premier conducteur (61a).

12. Applicateur de distribution de lumière (5) selon l'une des revendications 9 à 11, la section transversale du noyau (74) étant 0,8 à 1,2 fois la section transversale de la DEL (19).

13. Applicateur de distribution de lumière (5) selon l'une des revendications 9 à 12, le noyau et l'enveloppe formant une paire de conducteur aller et retour, le noyau et l'enveloppe étant isolés l'un de l'autre par une couche non conductrice électriquement qui est disposé entre le noyau et l'enveloppe.

14. Applicateur de distribution de lumière (5) selon l'une des revendications précédentes, la section transversale du premier conducteur (61a) étant au moins aussi grande que la section transversale de la DEL (19).

15. Applicateur de distribution de lumière (5) selon l'une des revendications précédentes, la DEL (19) étant en contact électrique et conducteur thermique avec une face frontale distale du premier conducteur (61a).
